# EUROPEAN PATENT APPLICATION

(11) **EP 3 928 799 A1**
(43) Date of publication of application: **29.12.2021**
(21) Application number: 20758455.8
(22) Date of filing: 20.02.2020
(51) Int. Cl.: A61K 48/00, C12N 15/113, A61K 31/711

(54) **OPTIMAL PS MODIFICATION PATTERN FOR HETERONUCLEIC ACIDS**

(30) Priority: 22.02.2019 JP 2019030635
(71) Applicant: National University Corporation Tokyo Medical and Dental University, Tokyo 113-8510 (JP)
(72) Inventor: YOKOTA, Takanori, Tokyo 113-8510 (JP); HARA, Rintaro, Tokyo 113-8510 (JP); NAGATA, Tetsuya, Tokyo 113-8510 (JP); KIZAWA, Hideki, Tokyo 100-0004 (JP); TAKAGI, Koh, Tokyo 100-0004 (JP)
(74) Representative: J A Kemp LLP
(86) International application number: PCT/JP2020/006660
(87) International publication number: WO 2020/171149

(57) **Abstract**

An object of the present invention is to provide a double-stranded nucleic acid complex having a reduced number of PS modifications in an antisense strand and maintaining an antisense effect. An optimal PS modification pattern for double-stranded nucleic acid complexes is disclosed. Such a nucleic acid complex comprising an antisense strand having an optimal PS modification pattern has a characteristic of having low toxicity and high bioavailability. The double-stranded nucleic acid complex comprising an antisense strand having an optimal PS modification pattern according to the present invention is useful when used as a nucleic acid drug that utilizes inhibition of expression of a specific gene (knockdown), activity that changes the function of coating or non-coding RNA for a specific gene, or activity that changes the function of RNA by inducing exon skipping during processing of pre-mRNA for a specific gene.

SEQUENCE LISTING

<110>  National University Corporation Tokyo Medical and Dental University

<120>  Optimum PS modification pattern of hetero nucleic acid

<130>  PH-8243-PCT

<150>  JP 2019-030635
<151>  2019-02-22

<160>  12

<170>  PatentIn version 3.5

<210>  1
<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>
<221>  misc_feature
<222>  (1)..(2), (2)..(3), (3)..(4), (4)..(5), (5)..(6), (6)..(7)
<223>  Phosphorothioate linkage

<220>
<221>  misc_feature
<222>  (7)..(8), (8)..(9), (9)..(10), (10)..(11), (11)..(12)
<223>  Phosphorothioate linkage

<220>
<221>  misc_feature
<222>  (12)..(13), (13)..(14)
<223>  Phosphorothioate linkage

<400>  1
tcatggctgc agct                                                    14


<210>  2
<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>
<221>  misc_feature
<222>  (1)..(2), (3)..(4), (4)..(5), (5)..(6), (6)..(7), (7)..(8)
<223>  Phosphorothioate linkage

<220>
<221>  misc_feature
<222>  (8)..(9), (9)..(10), (10)..(11), (11)..(12), (13)..(14)
<223>  Phosphorothioate linkage

<400>  2
tcatggctgc agct                                                          14


<210>  3
<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>
<221>  misc_feature
<222>  (1)..(2), (2)..(3), (9)..(10), (10)..(11), (11)..(12)
<223>  Phosphorothioate linkage

<220>
<221>  misc_feature
<222>  (12)..(13), (13)..(14)
<223>  Phosphorothioate linkage

<400>  3
tcatggctgc agct                                                          14


<210>  4

<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>
<221>  misc_feature
<222>  (1)..(2), (2)..(3), (3)..(4), (4)..(5), (5)..(6), (12)..(13)
<223>  Phosphorothioate linkage

<220>
<221>  misc_feature
<222>  (13)..(14)
<223>  Phosphorothioate linkage

<400>  4
tcatggctgc agct                                                          14


<210>  5
<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>
<221>  misc_feature
<222>  (1)..(2), (2)..(3), (5)..(6), (8)..(9), (11)..(12), (12)..(13)
<223>  Phosphorothioate linkage

<220>
<221>  misc_feature
<222>  (13)..(14)

<223>  Phosphorothioate linkage

<400>  5
tcatggctgc agct                                                          14


<210>  6
<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>
<221>  misc_feature
<222>  (1)..(2), (2)..(3), (3)..(4), (4)..(5), (6)..(7), (7)..(8)
<223>  Phosphorothioate linkage

<220>
<221>  misc_feature
<222>  (9)..(10), (10)..(11), (12)..(13), (13)..(14)
<223>  Phosphorothioate linkage

<400>  6
tcatggctgc agct                                                          14


<210>  7
<211>  14
<212>  DNA
<213>  Artificial

<220>
<223>  Synthetic

<220>
<223>  DNA/RNA molecule

<220>
<221>  misc_feature
<222>  (1)..(1), (14)..(14)
<223>  LNA

<220>
<221>  misc_feature
<222>  (2)..(2), (13)..(13)
<223>  5-methyl cytosine LNA

<220>

<221>  misc_feature
<222>  (1)..(2), (2)..(3), (4)..(5), (6)..(7), (8)..(9), (10)..(11)
<223>  Phosphorothioate linkage

<220>
<221>  misc_feature
<222>  (12)..(13), (13)..(14)
<223>  Phosphorothioate linkage

<400>  7
tcatggctgc agct                                                          14


<210>  8
<211>  14
<212>  RNA
<213>  Artificial

<220>
<223>  Synthetic

<220>
<221>  misc_feature
<222>  (1)..(1), (2)..(2), (13)..(13), (14)..(14)
<223>  2-o-Me RNA

<220>
<221>  misc_feature
<222>  (1)..(2), (2)..(3), (12)..(13), (13)..(14)
<223>  Phosphorothioate linkage

<400>  8
agcugcagcc auga                                                          14


<210>  9
<211>  16
<212>  DNA
<213>  Artificial

<220>
<223>  Synthetic

<220>
<223>  DNA/RNA molecule

<220>
<221>  misc_feature
<222>  (2)..(2), (3)..(3), (14)..(14), (15)..(15)
<223>  LNA

<220>
<221>  misc_feature
<222>  (1)..(1), (16)..(16)
<223>  5-methyl cytosine LNA

<220>
<221>  misc_feature
<222>  (1)..(2), (2)..(3), (3)..(4), (4)..(5), (5)..(6), (6)..(7)
<223>  Phosphorothioate linkage

<220>
<221>  misc_feature

<222>  (7)..(8), (8)..(9), (9)..(10), (10)..(11), (11)..(12)
<223>  Phosphorothioate linkage

<220>
<221>  misc_feature
<222>  (12)..(13), (13)..(14), (14)..(15), (16)..(16)
<223>  Phosphorothioate linkage

<400>  9
ctagttcact gaatgc                                                    16


<210>  10
<211>  16
<212>  RNA
<213>  Artificial

<220>
<223>  Synthetic

<220>
<221>  misc_feature
<222>  (1)..(1), (2)..(2), (3)..(3), (14)..(14), (15)..(15), (16)..(16)
<223>  2-o-Me RNA

<220>
<221>  misc_feature
<222>  (1)..(2), (2)..(3), (3)..(4), (13)..(14), (14)..(15), (15)..(16)
<223>  Phosphorothioate linkage

<400>  10
gcauucagug aacuag                                                    16


<210>  11
<211>  16
<212>  DNA
<213>  Artificial

<220>
<223>  Synthetic

<220>
<223>  DNA/RNA molecule

<220>
<221>  misc_feature
<222>  (2)..(2), (3)..(3), (15)..(15), (16)..(16)
<223>  LNA

<220>
<221>  misc_feature
<222>  (1)..(1), (13)..(13)
<223>  5-methyl cytosine LNA

<220>
<221>  misc_feature
<222>  (1)..(2), (2)..(3), (3)..(4), (4)..(5), (5)..(6), (6)..(7)
<223>  Phosphorothioate linkage

<220>
<221>  misc_feature
<222>  (7)..(8), (8)..(9), (9)..(10), (10)..(11), (11)..(12)

<223>  Phosphorothioate linkage

<220>
<221>  misc_feature
<222>  (12)..(13), (13)..(14), (14)..(15), (16)..(16)
<223>  Phosphorothioate linkage

<400>  11
cagcattggt attcag                                                       16


<210>  12
<211>  16
<212>  RNA
<213>  Artificial

<220>
<223>  Synthetic

<220>
<221>  misc_feature
<222>  (1)..(1), (2)..(2), (3)..(3), (14)..(14), (15)..(15), (16)..(16)
<223>  2-o-Me RNA

<220>
<221>  misc_feature
<222>  (1)..(2), (2)..(3), (3)..(4), (13)..(14), (14)..(15), (15)..(16)
<223>  Phosphorothioate linkage

<400>  12
cugaauacca augcug                                                       16

## Description

### Technical Field

The present invention relates to a structure which reduces side effects of a double-stranded nucleic acid having activity that inhibits or suppresses expression of a target gene by an antisense effect, and more particularly to a double-stranded nucleic acid in which an optimum PS modification pattern is formed in an antisense strand to enable both maintenance of the antisense effect and reduction of side effects such as toxicity.

### Background Art

In recent years, development of an oligonucleotide as a medicament referred to as a nucleic acid drug has been progressed, and in particular, from the viewpoint of high selectivity of a target gene, development of a nucleic acid drug with the utilization of an antisense method has been steadily progressed. The antisense method is a method in which an oligonucleotide complementary to a partial sequence of mRNA (sense strand) of a target gene (antisense oligonucleotide (ASO)) is introduced into cells to selectively inhibit or control expression of proteins encoded by the target gene. Examples of the single-stranded antisense oligonucleotide (hereinafter "ASO") include those having an action mechanism in which a double strand is formed by, for example, hybridization to target messenger RNA and a RNA strand is then degraded by RNase H to suppress expression of the target messenger RNA; and those that utilize an action mechanism in which ASO is hybridized to exon (encoding region) or intron (non-coding region) of target premessenger RNA to remove a portion that is spliced as an exon portion in the absence of ASO under a normal action by inducing exon skipping during processing of pre-mRNA to generate full-length mRNA.

ASO is required to have improved nuclease resistance for functioning as a nucleic acid drug. Patent Literature 1 describes that significant improvement of nuclease resistance can be achieved by replacing one of oxygen atoms of a phosphate group of a phosphodiester linkage, which is an inter-nucleic acid linkage of a natural oligonucleotide, with a sulfur atom to perform phosphorothioation (PS formation). However, it is known that as a nucleic acid drug, ASO containing an excessive number of phosphorothioated nucleic acids exhibits intense hepatotoxicity (Non Patent Literature 1 and Non Patent Literature 2) as a side effect which is not dependent on the sequence. Thus, it is required to improve nuclease resistance as compared to an oligomer with a phosphodiester linkage while suppressing a side effect manifesting as intense hepatoxicity, and improve activity of binding to target mRNA. As one means for solving this, it has been proposed that the inter-nucleic acid linkage of ASO has a configuration in which phosphodiester linkages (POs) and phosphorothioate linkages (PSs) are repeated alternately (Patent Literature 1).

Patent Literature 2 describes a nucleic acid drug which has a double strand including ASO having a sequence hybridizable to target messenger RNA and a nucleic acid strand complementary to the ASO, where the inter-nucleic acid linkage of the ASO has a structure in which phosphodiester linkages (POs) and phosphorothioate linkages (PSs) are repeated alternately (Patent Literature 2).

### Citation List

### Patent Literature

Patent Literature 1: WO00/03720A1
Patent Literature 2: WO2018/051762A1

### Non Patent Literature

Non Patent Literature 1: Nucleic Acids Res., 16, pp. 3209-3221 (1998)
Non Patent Literature 2: Trends Biotechnol. 1996 Oct; 14 (10): 376-87

### Summary of Invention

### Technical Problem

The present inventors recognize that a hetero-duplex oligonucleotide exhibits a high gene expression suppressing effect in various organs, but has problems including (1) a side effect as a tendency to bleed in the administration acute stage and (2) low bioavailability in subcutaneous administration.

It has been heretofore known that reduction in the number of PS modifications in an antisense strand leads to a decrease in side effects such as toxicity, but since the PS modification is essential for maintaining the stability of nucleic acids, reduction in the number of PSs destabilizes heteronucleic acids, resulting in impairment of effectivity.

An object of the present invention is to provide a double-stranded nucleic acid complex having a reduced number of PS modifications in an antisense strand (which means that side effects such as toxicity are reduced) and maintaining an antisense effect.

In addition, an object of the present invention is to provide a double-stranded nucleic acid complex having a reduced number of PS modifications in an antisense strand (which means that side effects such as toxicity are reduced) in a structure other than the previously proposed configuration with repeated PSs and POs and maintaining an antisense effect.

### Solution to Problem

The present inventors have found that when an antisense strand that is a first nucleotide of a double-stranded nucleic acid complex forms a PS modification pattern that is (POPOPS)n [PS represents a phosphorothioated PS-modified internucleotide linkage, PO represents a non-phosphorothioated natural internucleotide linkage, and n represents a natural number] or that has m number of contiguous PS-modified internucleotide linkages from the 5'-end to the 3'-end of the first oligonucleotide (m is a natural number equal to or less than 2/3, preferably equal to or less than 1/2, of the number of nucleic acids present in the first oligonucleotide), it is possible to obtain an effect of reducing a side effect as toxicity while maintaining an antisense effect.

Accordingly, the present invention relates to the following.
1. A double-stranded nucleic acid complex comprising: a first oligonucleotide comprising an antisense strand consisting of 7 to 30 nucleotides; and a second oligonucleotide having a sequence complementary to the first oligonucleotide and hybridized to the first oligonucleotide, wherein a modification pattern of internucleotide linkages in the first oligonucleotide comprises a pattern of (POPOPS)n with any internucleotide linkage added selected from PO, PS, POPO, PSPS, POPS and PSPO [PS represents a phosphorothioated PS-modified internucleotide linkage, PO represents a non-phosphorothioated natural internucleotide linkage, and n represents a natural number] or a pattern having m number of contiguous PS-modified internucleotide linkages from the 5'-end to the 3'-end of the first oligonucleotide (m is a natural number equal to or less than 2/3 of the number of nucleic acids present in the first oligonucleotide).
2. The double-stranded nucleic acid complex according to 1, comprising: a first oligonucleotide comprising an antisense strand consisting of 7 to 30 nucleotides; and a second oligonucleotide having a sequence complementary to the first oligonucleotide and hybridized to the first oligonucleotide, wherein the modification pattern of internucleotide linkages in the first oligonucleotide comprises a pattern of (POPOPS)n with any internucleotide linkage added selected from PO, PS, POPO, PSPS, POPS and PSPO [PS represents a phosphorothioated PS-modified internucleotide linkage, PO represents a non-phosphorothioated natural internucleotide linkage, and n is 1 to 9].
3. The double-stranded nucleic acid complex according to 2, wherein the modification pattern of internucleotide linkages in the first oligonucleotide is any of the following:
   POPOPS (n = 1)
   POPOPSPO
   POPOPSPS
   POPOPSPOPO
   POPOPSPSPS
   POPOPSPOPS
   POPOPSPSPO
   POPOPSPOPOPS (n = 2)
   POPOPSPOPOPSPO
   POPOPSPOPOPSPS
   POPOPSPOPOPSPOPO
   POPOPSPOPOPSPSPS
   POPOPSPOPOPSPOPS
   POPOPSPOPOPSPSPO
   POPOPSPOPOPSPOPOPS (n = 3)
   POPOPSPOPOPSPOPOPSPO
   POPOPSPOPOPSPOPOPSPS
   POPOPSPOPOPSPOPOPSPOPO
   POPOPSPOPOPSPOPOPSPSPS
   POPOPSPOPOPSPOPOPSPOPS
   POPOPSPOPOPSPOPOPSPSPO
   POPOPSPOPOPSPOPOPSPOPOPS (n = 4)
   POPOPSPOPOPSPOPOPSPOPOPSPO
   POPOPSPOPOPSPOPOPSPOPOPSPS
   POPOPSPOPOPSPOPOPSPOPOPSPOPO
   POPOPSPOPOPSPOPOPSPOPOPSPSPS
   POPOPSPOPOPSPOPOPSPOPOPSPOPS
   POPOPSPOPOPSPOPOPSPOPOPSPSPO
   POPOPSPOPOPSPOPOPSPOPOPSPOPOPS (n = 5)
   POPOPSPOPOPSPOPOPSPOPOPSPOPOPSPO
   POPOPSPOPOPSPOPOPSPOPOPSPOPOPSPS
   POPOPSPOPOPSPOPOPSPOPOPSPOPOPSPOPO
   POPOPSPOPOPSPOPOPSPOPOPSPOPOPSPSPS
   POPOPSPOPOPSPOPOPSPOPOPSPOPOPSPOPS
   POPOPSPOPOPSPOPOPSPOPOPSPOPOPSPSPO
   POPOPSPOPOPSPOPOPSPOPOPSPOPOPSPOPOPS (n = 6)
   POPOPSPOPOPSPOPOPSPOPOPSPOPOPSPOPOPSPO
   POPOPSPOPOPSPOPOPSPOPOPSPOPOPSPOPOPSPS
   POPOPSPOPOPSPOPOPSPOPOPSPOPOPSPOPOPSPOPO
   POPOPSPOPOPSPOPOPSPOPOPSPOPOPSPOPOPSPSPS
   POPOPSPOPOPSPOPOPSPOPOPSPOPOPSPOPOPSPOPS
   POPOPSPOPOPSPOPOPSPOPOPSPOPOPSPOPOPSPSPO
   POPOPSPOPOPSPOPOPSPOPOPSPOPOPSPOPOPSPOPOPS (n = 7)
   POPOPSPOPOPSPOPOPSPOPOPSPOPOPSPOPOPSPOPOPSPO
   POPOPSPOPOPSPOPOPSPOPOPSPOPOPSPOPOPSPOPOPSPS
   POPOPSPOPOPSPOPOPSPOPOPSPOPOPSPOPOPSPOPOPSPOPO
   POPOPSPOPOPSPOPOPSPOPOPSPOPOPSPOPOPSPOPOPSPSPS
   POPOPSPOPOPSPOPOPSPOPOPSPOPOPSPOPOPSPOPOPSPOPS
   POPOPSPOPOPSPOPOPSPOPOPSPOPOPSPOPOPSPOPOPSPSPO
   POPOPSPOPOPSPOPOPSPOPOPSPOPOPSPOPOPSPOPOPSPOPOPS (n = 8)
   POPOPSPOPOPSPOPOPSPOPOPSPOPOPSPOPOPSPOPOPSPOPOPSPO
   POPOPSPOPOPSPOPOPSPOPOPSPOPOPSPOPOPSPOPOPSPOPOPSPS
   POPOPSPOPOPSPOPOPSPOPOPSPOPOPSPOPOPSPOPOPSPOPOPSPOPO
   POPOPSPOPOPSPOPOPSPOPOPSPOPOPSPOPOPSPOPOPSPOPOPSPSPS
   POPOPSPOPOPSPOPOPSPOPOPSPOPOPSPOPOPSPOPOPSPOPOPSPOPS
   POPOPSPOPOPSPOPOPSPOPOPSPOPOPSPOPOPSPOPOPSPOPOPSPSPO
   POPOPSPOPOPSPOPOPSPOPOPSPOPOPSPOPOPSPOPOPSPOPOPSPOPOPS (n = 9)
   POPOPSPOPOPSPOPOPSPOPOPSPOPOPSPOPOPSPOPOPSPOPOPSPOPOPSPO
   POPOPSPOPOPSPOPOPSPOPOPSPOPOPSPOPOPSPOPOPSPOPOPSPOPOPSPS
   POPOPSPOPOPSPOPOPSPOPOPSPOPOPSPOPOPSPOPOPSPOPOPSPOPOPSPOPO
   POPOPSPOPOPSPOPOPSPOPOPSPOPOPSPOPOPSPOPOPSPOPOPSPOPOPSPSPS
   POPOPSPOPOPSPOPOPSPOPOPSPOPOPSPOPOPSPOPOPSPOPOPSPOPOPSPOPS
   POPOPSPOPOPSPOPOPSPOPOPSPOPOPSPOPOPSPOPOPSPOPOPSPOPOPSPSPO.
4. The double-stranded nucleic acid complex according to 3, wherein the modification pattern of internucleotide linkages in the first oligonucleotide is POPOPSPOPOPSPOPOPS.
5. A double-stranded nucleic acid complex comprising: a first oligonucleotide comprising an antisense strand consisting of 7 to 30 nucleotides; and a second oligonucleotide having a sequence complementary to the first oligonucleotide and hybridized to the first oligonucleotide, wherein a modification pattern of internucleotide linkages in the first oligonucleotide comprises a pattern having m number of contiguous PS-modified internucleotide linkages from the 5'-end to the 3'-end of the first oligonucleotide (m is a natural number equal to or less than 2/3 of the number of nucleic acids present in the first oligonucleotide).
6. A double-stranded nucleic acid complex comprising: a first oligonucleotide comprising an antisense strand consisting of 7 to 28 nucleotides; and a second oligonucleotide having a sequence complementary to the first oligonucleotide and hybridized to the first oligonucleotide, wherein a modification pattern of internucleotide linkages in the first oligonucleotide comprises a pattern having m number of contiguous PS-modified internucleotide linkages from the 5'-end to the 3'-end of the first oligonucleotide (m is a natural number equal to or less than 2/3 of the number of nucleic acids present in the first oligonucleotide, which is in the range of 1 to 13).
7. The double-stranded nucleic acid complex according to 6, wherein the modification pattern of internucleotide linkages in the first oligonucleotide is any of the following:
   PSPOPOPO
   PSPOPOPOPO
   PSPOPOPOPOPO
   PSPOPOPOPOPOPO
   PSPOPOPOPOPOPOPO
   PSPOPOPOPOPOPOPOPO
   PSPOPOPOPOPOPOPOPOPO
   PSPOPOPOPOPOPOPOPOPOPO
   PSPOPOPOPOPOPOPOPOPOPOPO
   PSPOPOPOPOPOPOPOPOPOPOPOPO
   PSPOPOPOPOPOPOPOPOPOPOPOPOPO
   PSPOPOPOPOPOPOPOPOPOPOPOPOPOPO
   PSPOPOPOPOPOPOPOPOPOPOPOPOPOPOPO
   PSPOPOPOPOPOPOPOPOPOPOPOPOPOPOPOPO
   PSPOPOPOPOPOPOPOPOPOPOPOPOPOPOPOPOPO
   PSPOPOPOPOPOPOPOPOPOPOPOPOPOPOPOPOPOPO
   PSPOPOPOPOPOPOPOPOPOPOPOPOPOPOPOPOPOPOPO
   PSPOPOPOPOPOPOPOPOPOPOPOPOPOPOPOPOPOPOPOPO
   PSPOPOPOPOPOPOPOPOPOPOPOPOPOPOPOPOPOPOPOPOPO
   PSPOPOPOPOPOPOPOPOPOPOPOPOPOPOPOPOPOPOPOPOPOPO
   PSPOPOPOPOPOPOPOPOPOPOPOPOPOPOPOPOPOPOPOPOPOPOPO
   PSPOPOPOPOPOPOPOPOPOPOPOPOPOPOPOPOPOPOPOPOPOPOPOPO
   PSPOPOPOPOPOPOPOPOPOPOPOPOPOPOPOPOPOPOPOPOPOPOPOPOPO
   PSPOPOPOPOPOPOPOPOPOPOPOPOPOPOPOPOPOPOPOPOPOPOPOPOPOPO
   PSPSPOPO
   PSPSPOPOPO
   PSPSPOPOPOPO
   PSPSPOPOPOPOPO
   PSPSPOPOPOPOPOPO
   PSPSPOPOPOPOPOPOPO
   PSPSPOPOPOPOPOPOPOPO
   PSPSPOPOPOPOPOPOPOPOPO
   PSPSPOPOPOPOPOPOPOPOPOPO
   PSPSPOPOPOPOPOPOPOPOPOPOPO
   PSPSPOPOPOPOPOPOPOPOPOPOPOPO
   PSPSPOPOPOPOPOPOPOPOPOPOPOPOPO
   PSPSPOPOPOPOPOPOPOPOPOPOPOPOPOPO
   PSPSPOPOPOPOPOPOPOPOPOPOPOPOPOPOPO
   PSPSPOPOPOPOPOPOPOPOPOPOPOPOPOPOPOPO
   PSPSPOPOPOPOPOPOPOPOPOPOPOPOPOPOPOPOPO
   PSPSPOPOPOPOPOPOPOPOPOPOPOPOPOPOPOPOPOPO
   PSPSPOPOPOPOPOPOPOPOPOPOPOPOPOPOPOPOPOPOPO
   PSPSPOPOPOPOPOPOPOPOPOPOPOPOPOPOPOPOPOPOPOPO
   PSPSPOPOPOPOPOPOPOPOPOPOPOPOPOPOPOPOPOPOPOPOPO
   PSPSPOPOPOPOPOPOPOPOPOPOPOPOPOPOPOPOPOPOPOPOPOPO
   PSPSPOPOPOPOPOPOPOPOPOPOPOPOPOPOPOPOPOPOPOPOPOPOPO
   PSPSPOPOPOPOPOPOPOPOPOPOPOPOPOPOPOPOPOPOPOPOPOPOPOPO
   PSPSPOPOPOPOPOPOPOPOPOPOPOPOPOPOPOPOPOPOPOPOPOPOPOPOPO
   PSPSPSPOPOPO
   PSPSPSPOPOPOPO
   PSPSPSPOPOPOPOPO
   PSPSPSPOPOPOPOPOPO
   PSPSPSPOPOPOPOPOPOPO
   PSPSPSPOPOPOPOPOPOPOPO
   PSPSPSPOPOPOPOPOPOPOPOPO
   PSPSPSPOPOPOPOPOPOPOPOPOPO
   PSPSPSPOPOPOPOPOPOPOPOPOPOPO
   PSPSPSPOPOPOPOPOPOPOPOPOPOPOPO
   PSPSPSPOPOPOPOPOPOPOPOPOPOPOPOPO
   PSPSPSPOPOPOPOPOPOPOPOPOPOPOPOPOPO
   PSPSPSPOPOPOPOPOPOPOPOPOPOPOPOPOPOPO
   PSPSPSPOPOPOPOPOPOPOPOPOPOPOPOPOPOPOPO
   PSPSPSPOPOPOPOPOPOPOPOPOPOPOPOPOPOPOPOPO
   PSPSPSPOPOPOPOPOPOPOPOPOPOPOPOPOPOPOPOPOPO
   PSPSPSPOPOPOPOPOPOPOPOPOPOPOPOPOPOPOPOPOPOPO
   PSPSPSPOPOPOPOPOPOPOPOPOPOPOPOPOPOPOPOPOPOPOPO
   PSPSPSPOPOPOPOPOPOPOPOPOPOPOPOPOPOPOPOPOPOPOPOPO
   PSPSPSPOPOPOPOPOPOPOPOPOPOPOPOPOPOPOPOPOPOPOPOPOPO
   PSPSPSPOPOPOPOPOPOPOPOPOPOPOPOPOPOPOPOPOPOPOPOPOPOPO
   PSPSPSPOPOPOPOPOPOPOPOPOPOPOPOPOPOPOPOPOPOPOPOPOPOPOPO
   PSPSPSPSPOPOPOPO
   PSPSPSPSPOPOPOPOPO
   PSPSPSPSPOPOPOPOPOPO
   PSPSPSPSPOPOPOPOPOPOPO
   PSPSPSPSPOPOPOPOPOPOPOPO
   PSPSPSPSPOPOPOPOPOPOPOPOPO
   PSPSPSPSPOPOPOPOPOPOPOPOPOPO
   PSPSPSPSPOPOPOPOPOPOPOPOPOPOPO
   PSPSPSPSPOPOPOPOPOPOPOPOPOPOPOPO
   PSPSPSPSPOPOPOPOPOPOPOPOPOPOPOPOPO
   PSPSPSPSPOPOPOPOPOPOPOPOPOPOPOPOPOPO
   PSPSPSPSPOPOPOPOPOPOPOPOPOPOPOPOPOPOPO
   PSPSPSPSPOPOPOPOPOPOPOPOPOPOPOPOPOPOPOPO
   PSPSPSPSPOPOPOPOPOPOPOPOPOPOPOPOPOPOPOPOPO
   PSPSPSPSPOPOPOPOPOPOPOPOPOPOPOPOPOPOPOPOPOPO
   PSPSPSPSPOPOPOPOPOPOPOPOPOPOPOPOPOPOPOPOPOPOPO
   PSPSPSPSPOPOPOPOPOPOPOPOPOPOPOPOPOPOPOPOPOPOPOPO
   PSPSPSPSPOPOPOPOPOPOPOPOPOPOPOPOPOPOPOPOPOPOPOPOPO
   PSPSPSPSPOPOPOPOPOPOPOPOPOPOPOPOPOPOPOPOPOPOPOPOPOPO
   PSPSPSPSPOPOPOPOPOPOPOPOPOPOPOPOPOPOPOPOPOPOPOPOPOPOPO
   PSPSPSPSPSPOPOPOPOPO
   PSPSPSPSPSPOPOPOPOPOPO
   PSPSPSPSPSPOPOPOPOPOPOPO
   PSPSPSPSPSPOPOPOPOPOPOPOPO
   PSPSPSPSPSPOPOPOPOPOPOPOPOPO
   PSPSPSPSPSPOPOPOPOPOPOPOPOPOPO
   PSPSPSPSPSPOPOPOPOPOPOPOPOPOPOPO
   PSPSPSPSPSPOPOPOPOPOPOPOPOPOPOPOPO
   PSPSPSPSPSPOPOPOPOPOPOPOPOPOPOPOPOPO
   PSPSPSPSPSPOPOPOPOPOPOPOPOPOPOPOPOPOPO
   PSPSPSPSPSPOPOPOPOPOPOPOPOPOPOPOPOPOPOPO
   PSPSPSPSPSPOPOPOPOPOPOPOPOPOPOPOPOPOPOPOPO
   PSPSPSPSPSPOPOPOPOPOPOPOPOPOPOPOPOPOPOPOPOPO
   PSPSPSPSPSPOPOPOPOPOPOPOPOPOPOPOPOPOPOPOPOPOPO
   PSPSPSPSPSPOPOPOPOPOPOPOPOPOPOPOPOPOPOPOPOPOPOPO
   PSPSPSPSPSPOPOPOPOPOPOPOPOPOPOPOPOPOPOPOPOPOPOPOPO
   PSPSPSPSPSPOPOPOPOPOPOPOPOPOPOPOPOPOPOPOPOPOPOPOPOPO
   PSPSPSPSPSPOPOPOPOPOPOPOPOPOPOPOPOPOPOPOPOPOPOPOPOPOPO
   PSPSPSPSPSPSPOPOPOPOPOPOPO
   PSPSPSPSPSPSPOPOPOPOPOPOPOPO
   PSPSPSPSPSPSPOPOPOPOPOPOPOPOPO
   PSPSPSPSPSPSPOPOPOPOPOPOPOPOPOPO
   PSPSPSPSPSPSPOPOPOPOPOPOPOPOPOPOPO
   PSPSPSPSPSPSPOPOPOPOPOPOPOPOPOPOPOPO
   PSPSPSPSPSPSPOPOPOPOPOPOPOPOPOPOPOPOPO
   PSPSPSPSPSPSPOPOPOPOPOPOPOPOPOPOPOPOPOPO
   PSPSPSPSPSPSPOPOPOPOPOPOPOPOPOPOPOPOPOPOPO
   PSPSPSPSPSPSPOPOPOPOPOPOPOPOPOPOPOPOPOPOPOPO
   PSPSPSPSPSPSPOPOPOPOPOPOPOPOPOPOPOPOPOPOPOPOPO
   PSPSPSPSPSPSPOPOPOPOPOPOPOPOPOPOPOPOPOPOPOPOPOPO
   PSPSPSPSPSPSPOPOPOPOPOPOPOPOPOPOPOPOPOPOPOPOPOPOPO
   PSPSPSPSPSPSPOPOPOPOPOPOPOPOPOPOPOPOPOPOPOPOPOPOPOPO
   PSPSPSPSPSPSPOPOPOPOPOPOPOPOPOPOPOPOPOPOPOPOPOPOPOPOPO
   PSPSPSPSPSPSPSPOPOPOPOPOPOPOPO
   PSPSPSPSPSPSPSPOPOPOPOPOPOPOPOPO
   PSPSPSPSPSPSPSPOPOPOPOPOPOPOPOPOPO
   PSPSPSPSPSPSPSPOPOPOPOPOPOPOPOPOPOPO
   PSPSPSPSPSPSPSPOPOPOPOPOPOPOPOPOPOPOPO
   PSPSPSPSPSPSPSPOPOPOPOPOPOPOPOPOPOPOPOPO
   PSPSPSPSPSPSPSPOPOPOPOPOPOPOPOPOPOPOPOPOPO
   PSPSPSPSPSPSPSPOPOPOPOPOPOPOPOPOPOPOPOPOPOPO
   PSPSPSPSPSPSPSPOPOPOPOPOPOPOPOPOPOPOPOPOPOPOPO
   PSPSPSPSPSPSPSPOPOPOPOPOPOPOPOPOPOPOPOPOPOPOPOPO
   PSPSPSPSPSPSPSPOPOPOPOPOPOPOPOPOPOPOPOPOPOPOPOPOPO
   PSPSPSPSPSPSPSPOPOPOPOPOPOPOPOPOPOPOPOPOPOPOPOPOPOPO
   PSPSPSPSPSPSPSPOPOPOPOPOPOPOPOPOPOPOPOPOPOPOPOPOPOPOPO
   PSPSPSPSPSPSPSPSPOPOPOPOPOPOPOPOPO
   PSPSPSPSPSPSPSPSPOPOPOPOPOPOPOPOPOPO
   PSPSPSPSPSPSPSPSPOPOPOPOPOPOPOPOPOPOPO
   PSPSPSPSPSPSPSPSPOPOPOPOPOPOPOPOPOPOPOPO
   PSPSPSPSPSPSPSPSPOPOPOPOPOPOPOPOPOPOPOPOPO
   PSPSPSPSPSPSPSPSPOPOPOPOPOPOPOPOPOPOPOPOPOPO
   PSPSPSPSPSPSPSPSPOPOPOPOPOPOPOPOPOPOPOPOPOPOPO
   PSPSPSPSPSPSPSPSPOPOPOPOPOPOPOPOPOPOPOPOPOPOPOPO
   PSPSPSPSPSPSPSPSPOPOPOPOPOPOPOPOPOPOPOPOPOPOPOPOPO
   PSPSPSPSPSPSPSPSPOPOPOPOPOPOPOPOPOPOPOPOPOPOPOPOPOPO
   PSPSPSPSPSPSPSPSPOPOPOPOPOPOPOPOPOPOPOPOPOPOPOPOPOPOPO
   PSPSPSPSPSPSPSPSPSPOPOPOPOPOPOPOPOPOPO
   PSPSPSPSPSPSPSPSPSPOPOPOPOPOPOPOPOPOPOPO
   PSPSPSPSPSPSPSPSPSPOPOPOPOPOPOPOPOPOPOPOPO
   PSPSPSPSPSPSPSPSPSPOPOPOPOPOPOPOPOPOPOPOPOPO
   PSPSPSPSPSPSPSPSPSPOPOPOPOPOPOPOPOPOPOPOPOPOPO
   PSPSPSPSPSPSPSPSPSPOPOPOPOPOPOPOPOPOPOPOPOPOPOPO
   PSPSPSPSPSPSPSPSPSPOPOPOPOPOPOPOPOPOPOPOPOPOPOPOPO
   PSPSPSPSPSPSPSPSPSPOPOPOPOPOPOPOPOPOPOPOPOPOPOPOPOPO
   PSPSPSPSPSPSPSPSPSPOPOPOPOPOPOPOPOPOPOPOPOPOPOPOPOPOPO
   PSPSPSPSPSPSPSPSPSPSPOPOPOPOPOPOPOPOPOPOPO
   PSPSPSPSPSPSPSPSPSPSPOPOPOPOPOPOPOPOPOPOPOPO
   PSPSPSPSPSPSPSPSPSPSPOPOPOPOPOPOPOPOPOPOPOPOPO
   PSPSPSPSPSPSPSPSPSPSPOPOPOPOPOPOPOPOPOPOPOPOPOPO
   PSPSPSPSPSPSPSPSPSPSPOPOPOPOPOPOPOPOPOPOPOPOPOPOPO
   PSPSPSPSPSPSPSPSPSPSPOPOPOPOPOPOPOPOPOPOPOPOPOPOPOPO
   PSPSPSPSPSPSPSPSPSPSPOPOPOPOPOPOPOPOPOPOPOPOPOPOPOPOPO
   PSPSPSPSPSPSPSPSPSPSPSPOPOPOPOPOPOPOPOPOPOPOPO
   PSPSPSPSPSPSPSPSPSPSPSPOPOPOPOPOPOPOPOPOPOPOPOPO
   PSPSPSPSPSPSPSPSPSPSPSPOPOPOPOPOPOPOPOPOPOPOPOPOPO
   PSPSPSPSPSPSPSPSPSPSPSPOPOPOPOPOPOPOPOPOPOPOPOPOPOPO
   PSPSPSPSPSPSPSPSPSPSPSPOPOPOPOPOPOPOPOPOPOPOPOPOPOPOPO
   PSPSPSPSPSPSPSPSPSPSPSPSPOPOPOPOPOPOPOPOPOPOPOPOPO
   PSPSPSPSPSPSPSPSPSPSPSPSPOPOPOPOPOPOPOPOPOPOPOPOPOPO
   PSPSPSPSPSPSPSPSPSPSPSPSPOPOPOPOPOPOPOPOPOPOPOPOPOPOPO
   PSPSPSPSPSPSPSPSPSPSPSPSPSPOPOPOPOPOPOPOPOPOPOPOPOPOPO.
8. The double-stranded nucleic acid complex according to 7, wherein the modification pattern of internucleotide linkages in the first oligonucleotide is PsPsPsPOPOPOPOPOPO.
9. The double-stranded nucleic acid complex according to any one of 1 to 8, wherein the first oligonucleotide includes a gap region and a wing region located at each of both ends of the gap region, and the modification pattern of internucleotide linkages in the first oligonucleotide is a modification pattern of internucleotide linkages in the gap region.
10. The double-stranded nucleic acid complex according to any one of 1 to 9, wherein a ligand is bound to the second oligonucleotide.
11. The double-stranded nucleic acid complex according to any one of 1 to 10, wherein the wing region of the first oligonucleotide includes 1 to 4 nucleic acids.
12. The double-stranded nucleic acid complex according to any one of 1 to 11, wherein the first oligonucleotide has a structure represented by (first wing region)r-(gap region)s-(second wing region)r [r is 1 to 10 and s is 4 to 28].
13. The double-stranded nucleic acid complex according to any one of 1 to 12, wherein the wing region of the first oligonucleotide comprises one or more PS-modified internucleotide linkages.
14. The double-stranded nucleic acid complex according to any one of 1 to 12, wherein all the internucleotide linkages in the wing region of the first oligonucleotide are PS-modified internucleotide linkages.
15. The double-stranded nucleic acid complex according to any one of 1 to 12, wherein the internucleotide linkage on the 5'-end of a first wing region of the first oligonucleotide and the internucleotide linkage on the 3'-end of a second wing region are PS-modified internucleotide linkages.
16. The double-stranded nucleic acid complex according to any one of 1 to 15, wherein the gap region of the first oligonucleotide includes DNA or a DNA-modified nucleotide, and the second oligonucleotide includes RNA or a RNA-modified nucleotide.

The present description encompasses the disclosure of JP Patent Application No. 2019-030635, based on which the priority of the present application is claimed.

### Advantageous Effects of Invention

Since in a double-stranded nucleic acid complex, an antisense strand that is a first oligonucleotide has a PS modification pattern that is (POPOPS)n [PS represents a phosphorothioated PS-modified internucleotide linkage, PO represents a non-phosphorothioated natural internucleotide linkage, and n represents a natural number] or that has m number of contiguous PS-modified internucleotide linkages from the 5'-end to the 3'-end of the first oligonucleotide (m is a natural number equal to or less than 2/3, preferably equal to or less than 1/2, of the number of nucleic acids present in the first oligonucleotide), it is possible to obtain an effect of reducing a side effect as toxicity while maintaining an antisense effect.

In addition, according to the present invention, it is possible to avoid a side effect as a tendency to bleed in the administration acute stage and achieve improvement in bioavailability in subcutaneous administration.

### Brief Description of Drawings

[Figure 1] Figure 1 is a diagram showing a comparison of knockdown action in the liver when 7 types of PTEN HDOs (14 mer) having different PS modification patterns for antisense strands are administered to a mouse (n = 4).
[Figure 2] Figure 2 is a diagram showing changes in mouse liver weight when 7 types of PTEN HDOs (14 mer) having different PS modification patterns for antisense strands are administered (n = 4).
[Figure 3] Figure 3 is a diagram showing changes in blood ALT activity when 7 types of PTEN HDOs (14 mer) having different PS modification patterns for antisense strands are administered to a mouse (n = 4).
[Figure 4] Figure 4 is a diagram showing the results of comparison of knockdown action in the mouse liver by two types of MALAT1 HDOs (16 mer) having different PS modification patterns.
[Figure 5] Figure 5 is a diagram showing changes in blood ALT activity when MALAT1 HDOs having different numbers of PS modifications are administered.
[Figure 6] Figure 6 is a diagram showing the results of comparison of knockdown action in the mouse liver by two types of MALAT1 HDOs (16 mer) having different PS modification patterns.
[Figure 7] Figure 7 is a diagram showing changes in blood ALT activity when ApoB HDOs having different numbers of PS modifications are administered.

### Description of Embodiments

The present invention provides a double-stranded nucleic acid complex comprising a first oligonucleotide, and a second oligonucleotide having a sequence complementary to the first oligonucleotide, wherein the first oligonucleotide and the second oligonucleotide are hybridized to each other, wherein the first oligonucleotide sequence is (POPOPS)n (n is a natural number), or has m number of contiguous PS-modified nucleotides linkages from the 5'-end to the 3'-end of the first oligonucleotide (m is a natural number equal to or less than 2/3, preferably equal to or less than 1/2, of the number of nucleic acids present in the first oligonucleotide).

The first oligonucleotide is an antisense nucleic acid comprising an antisense strand, and has an antisense effect. The first oligonucleotide is called an antisense strand, and the second oligonucleotide is called a sense strand.

The antisense nucleic acid is a nucleic acid complementary to a target transcription product such as a target gene transcription product, and has an antisense effect on a target gene. The "antisense effect" means suppression of expression of a target gene or a target transcription product level which is caused by hybridization of a target transcription product (RNA sense strand) to, for example, a DNA strand complementary to a partial sequence of the target transcription product, a strand normally designed to produce an antisense effect, or the like. In certain cases, the antisense effect means inhibition of translation which can be caused by covering the transcription product with a hybridization product or a splicing function converting effect such as exon skipping, and/or the suppression that occurs due to degradation of the transcription product which can be caused by recognition of a hybridized portion.

The "target gene" or "target transcription product", whose expression is suppressed by the antisense effect, is not particularly limited, and examples thereof include genes whose expression is elevated in various diseases. The "target gene transcription product" is mRNA transcripted from genome DNA that encodes a target gene, and includes mRNA which has not been subjected to modification with a base, and mRNA precursors which have not been subjected to splicing. Normally, the "transcription product" may be any RNA that is synthesized by DNA-dependent RNA polymerase.

The term "complementary" means a relationship which enables formation of a so-called Watson-Crick base pair (natural base pair) or non-Watson-Crick base pair (e.g. Hoogsteen base pair) via a hydrogen bond here. When a sufficient number of nucleic acid bases of the antisense strand can be hydrogen-bonded to corresponding nucleic acid bases of the target nucleic acid, the antisense strand and the target nucleic acid are complementary to each other. A non-complementary nucleic acid base between the antisense strand and the target nucleic acid can be accepted under the condition that the antisense strand can be hybridized specifically to a target nucleic acid. Further, an antisense compound can be hybridized to one or more segments of a desired nucleic acid, and accordingly, intervening or adjacent segments (e.g. loop structures, mismatch or hairpin structures) are not involved in the hybridization event.

In a certain embodiment, an antisense strand, which is a first oligonucleotide, is complementary to a target nucleic acid by 80 to 100%, preferably 90 to 100%, more preferably 95 to 100%, 96 to 100%, 97 to 100%, 98 to 100%, 99 to 100% or 100%. The ratio of complementarity of the antisense strand to the target nucleic acid can be determined using a conventional method.

For example, 16 of 20 nucleic acid bases of the antisense strand are complementary to the target region, and therefore the specifically hybridized antisense strand has complementarity at 80 percent. In this example, complementary nucleic acid bases can collect or can scatter at the remaining non-complementary nucleic acid bases, and these non-complementary nucleic acid bases are not required to be adjacent one another or to the complementary nucleic acid bases.

In the present description, the "nucleic acid" refers to a natural nucleic acid, a non-natural nucleic acid and/or a nucleic acid analog, and the "nucleic acid" may mean a monomer nucleotide, or mean an oligonucleotide or polynucleotide including a plurality of monomers. Here, the term "nucleic acid strand" is also used for referring to an oligonucleotide or polynucleotide. The whole or a part of the nucleic acid strand may be prepared by a chemical synthesis method involving, for example, use of an automatic synthesis machine, or may be prepared by enzyme treatment using, without limitation, polymerase, ligase or a restriction enzyme reaction. In one embodiment of the present invention, the polynucleotide in the antisense nucleic acid (and the polynucleotide in the complementary strand) according to the present invention is one artificially prepared by chemical synthesis or an enzymatic reaction.

In the present description, the "nucleotide" means a compound in which a sugar portion of a nucleoside forms an ester with phosphoric acid, where the "nucleoside" means a glycoside compound in which an organic base containing nitrogen, such as a purine base or a pyrimidine base, is bound to a reducing group of a sugar through a glycoside linkage.

The "deoxyribonucleotide" is a nucleotide with a sugar portion including D-2-deoxyribose, and is also referred to as a "DNA nucleotide". The "ribonucleotide" is a nucleotide with a sugar portion including D-ribose, and is also referred to as an "RNA nucleotide". The "deoxyribonucleoside" is a nucleoside with a sugar portion including D-2-deoxyribose, and is also referred to as a "DNA nucleotide". The "ribonucleoside" is a nucleoside with a sugar portion including D-ribose, and is also referred to as a "RNA nucleoside".

The "oligonucleotide" means a chain substance having a nucleotide as a basic unit and obtained by polymerization of a plurality of nucleotides which are connected with phosphoric acid forming a bridge of diester linkage between 3'- and 5'-position carbon atoms of a sugar between the nucleotides. The oligonucleotide may be referred to as a polynucleotide. In the present description, the nucleotide (in a polymerized state) present in such an oligonucleotide as its constituent unit may be also referred to as a "nucleotide".

In the present description, the "natural nucleic acid" refers to a naturally occurring polynucleotide having a nucleotide as a basic unit and obtained by polymerization of nucleotides which are connected with phosphoric acid forming a bridge of diester linkage between 3'- and 5'-position carbon atoms of a sugar between the nucleotides. Normally, RNA in which ribonucleotides having any of the bases of adenine, guanine, cytosine and uracil are connected and/or DNA in which deoxyribonucleotides having any of the bases of adenine, guanine, cytosine and thymine correspond to the natural nucleic acid. In the present description, the linkage between the nucleotides is also referred to as an "inter-nucleic acid linkage".

In the present description, the "non-natural nucleic acid" includes or consists of non-natural nucleotides. Here, the "non-natural nucleotide" is an artificially constructed or artificially chemically modified nucleotide which does not naturally occur, where the nucleotide is similar in properties and/or structure to the naturally occurring nucleotide, or contains a nucleoside or a base similar in properties and/or structure to a naturally occurring nucleoside or base. Examples thereof include nucleotides having abasic nucleosides, arabinonucleosides, 2'-deoxyuridine, α-deoxyribonucleosides, β-L-deoxyribonucleosides, and having other sugar modifications. Further, examples thereof include nucleotides having sugar modifications including substituted pentoses (2'-O-methylribose, 2'-deoxy-2'-fluororibose, 3'-O-methylribose and 1',2'-deoxyribose), arabinose, substituted arabinose sugars, substituted hexoses and alfa-anomers. The non-natural nucleotide also includes nucleotides including artificially constructed base analogs or artificially chemically modified bases (modified bases). Examples of the modification include 5-methylation, 5-fluorination, 5-bromination, 5-iodation and N4-methylation of cytosine; 5-demethylation, 5-fluorination, 5-bromination and 5-iodation of thymidine; N6-methylation and 8-bromination of adenine; N2-methylation and 8-bromination of guanine; and methylphosphonation, methylthiophosphonation, chiral-methylphosphonation, phosphorodithioation, phosphoroamidatation, 2'-O-methylation, 2'-methoxyethyl (MOE) ation, 2'-aminopropyl (AP) ation and 2'-fluorination. Examples of the "base analog" include a 2-oxo(1H)-pyridin-3-yl group, a 5-position-substituted 2-oxo(1H)-pyridin-3-yl group, a 2-amino-6-(2-thiazolyl)purin-9-yl group, a 2-amino-6-(2-thiazolyl)purin-9-yl group, and a 2-amino-6-(2-oxazolyl)purin-9-yl group. Examples of the "modified base" include modified pyrimidines (e.g. 5-hydroxycytosine, 5-fluorouracil and 4-thiouracil), modified purines (e.g. 6-methyladenine and 6-thioguanosine) and other heterocyclic bases. The non-natural nucleotide may also include modified nucleotides and nucleic acid analogs such as methylphosphonate-type DNA/RNA, phosphorothioate-type DNA/RNA (i.e. DNA/RNA having a thiophosphate linkage), phosphoramidate-type DNA/RNA and 2'-O-methyl-type DNA/RNA. However, for the first oligonucleotide, phosphorothioated internucleotide linkages are present in a certain pattern. The pattern in which the phosphorothioated internucleotide linkages are present will be described later. The non-natural nucleotide includes nucleotide analogs which are artificially constructed compounds similar in structure and/or properties to natural nucleic acids. The "nucleotide analog" is any nucleic acid in which affinity to a complementary strand or a partial sequence of a target transcription product and/or resistance of the nucleic acid to a nuclease is enhanced by a modification (e.g. a bridge group or a substituent), and examples thereof may include peptide nucleic acids (PNAs), peptide nucleic acids having a phosphate group (PHONAs), bridged nucleic acids/locked nucleic acids (BNAs/LNAs), morpholino nucleic acids, hexitol nucleic acids (HNAs), cyclohexene nucleic acids (CeNAs), glycol nucleic acids (GNAs), threose nucleic acids (TNAs), tricyclo-DNAs (tcDNAs), 2'-O-methylated nucleic acids, 2'-MOE (2'-O-methoxyethyl)ated nucleic acids, 2'-AP(2'-O-aminopropyl)ated nucleic acids, 2'-fluorinated nucleic acids, 2'F-arabinonucleic acids (2'-F-ANA) and basic polyamino acids (Poly L-Lys, Poly L-Arg). In the present description, these nucleic acids are also referred to collectively as "modified nucleotides". The modified nucleotide and the modified nucleotide analog are not limited to the modified nucleotides and the modified nucleotide analogs exemplified in the present description.

In an embodiment, the nucleotide analog is LNA represented by the following formula (1). 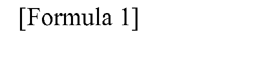 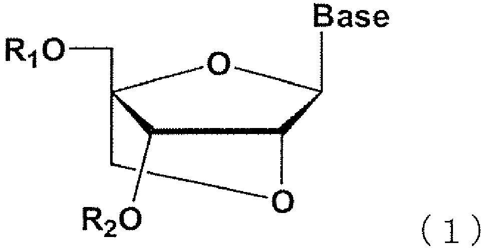 (In Formula (1), Base represents an aromatic heterocyclic group or an aromatic hydrocarbon ring group optionally having a substituent, for example a base site (purine base or pyrimidine base) of a natural nucleoside, or a base site of a non-natural (modified) nucleoside. Examples of the modification of the base site are as described above.

R₁ and R₂ may be the same or different, and each represent a hydrogen atom, a protective group for hydroxyl groups for synthesis of nucleic acids, an alkyl group, an alkenyl group, a cycloalkyl group, an aryl group, an aralkyl group, an acyl group, a sulfonyl group, a silyl group, a phosphate group, a phosphate group protected with a protective group for synthesis of nucleic acids, or -P(R₄)R₅ [where R₄ and R₅ may be the same or different, and each represent a hydroxyl group, a hydroxyl group protected with a protective group for synthesis of nucleic acids, a mercapto group, a mercapto group protected with a protective group for synthesis of nucleic acids, an amino group, an alkoxy group having 1 to 5 carbon atoms, an alkylthio group having 1 to 5 carbon atoms, a cyanoalkoxy group having 1 to 6 carbon atoms, or an amino group substituted with an alkyl group having 1 to 5 carbon atoms.])

The compound represented by the formula is a nucleoside, and "LNA" and normally "BNA" in an embodiment include a form (nucleotide) in which a phosphate group is bound to the nucleoside. That is, BNA such as LNA is incorporated as a nucleotide in a nucleic acid strand containing a double-stranded nucleic acid complex.

In a certain embodiment, the oligonucleotide contains modified nucleotides. In an embodiment, as long as the oligonucleotide containing an antisense strand has a certain pattern in which phosphorothioated internucleotide linkages are present, the oligonucleotide may contain nucleotides and optionally modified nucleotides, or may contain only modified nucleotides. For preventing cleavage of the nucleic acid strand by the digestive action of an enzyme *in vivo*, the antisense oligonucleotide may contain any number of modified nucleotides at any location for the purpose of making the inter-nucleic acid linkage stronger than that of natural nucleic acids.

The length of the first oligonucleotide in the double-stranded nucleic acid complex of the present invention is not limited, and is 5 to 100 nucleotides, preferably 7 to 50 nucleotides, more preferably 7 to 30 nucleotides, still more preferably 7 to 25 nucleotides, even more preferably 7 to 20 nucleotides.

The length of the second oligonucleotide is not limited as long as it is annealed with the first oligonucleotide, and the length of the second oligonucleotide may be the same as or different from the length of the first oligonucleotide, and is preferably the same as the length of the first oligonucleotide.

Preferably, the first oligonucleotide contains a gap region, a 5'-wing region connected to the 5'-end of the gap region, and a 3'-wing region connected to the 3'-end of the gap region. That is, the first oligonucleotide has wing regions at both ends of the gap region, and are composed of these regions. Preferably, the wing region is disposed at the 5'-end and/or the 3'-end of the antisense nucleic acid. The gap region is also referred to as a central nucleotide region. The 5'-wing region may be referred to as a first wing region, and the 3'-wing region may be referred to as a second wing region.

The gap region contains 4 or more continuous nucleotides or modified nucleotides which can be recognized by RNAse H, preferably 4 or more continuous DNA nucleotides or modified DNA nucleotides. The length of the gap region is 4 to 28 bases, preferably 4 to 20 bases, more preferably 5 to 16 bases, still more preferably 6 to 16 bases.

The 5'-wing region and the 3'-wing region are independent of each other, and may contain at least one modified nucleotide. Examples of the preferred modified nucleotide include LNAs, and all the nucleotides of the 5'-wing region and the 3'-wing region may be LNAs. In the first oligonucleotide, DNA having a strand length of at least 4 bases is disposed at the center, and LNA (or another BNA) having a high binding capability with RNA (i.e. target transcription product) is disposed on each of both ends, whereby such a complex strand promotes cleavage of target RNA by RNaseH.

The lengths of the 5'-wing region and the 3'-wing region are each independently 1 to 10 bases, preferably 1 to 7 bases, more preferably 1 to 4 bases, still more preferably 2 to 4 bases, or 2 to 5 bases.

The wing region is a segment in which chemically modified nucleotides are disposed for enhancing mainly the nuclease resistance of the antisense nucleic acid and the affinity of such a nucleic acid to mRNA as a target.

In one aspect of the present invention, the first oligonucleotide containing an antisense strand has a sequence represented by (first wing region)r-(gap region)s-(second wing region)r. Here, the first wing region is a 5'-wing region, and the second wing region is a 3'-wing region. In addition, r and s represent the number of bases forming the wing region and the gap region, respectively, and r is 1 to 10, preferably 1 to 7, more preferably 1 to 4, still more preferably 2 to 4, or 2 to 5 or 1 to 4. s is 4 to 28, preferably 4 to 20, more preferably 5 to 16, still more preferably 6 to 16.

The second oligonucleotide is an oligonucleotide including at least one nucleic acid selected from the group consisting of RNA, DNA, PNA (peptide nucleic acid) and BNA (e.g. LNA). When the second oligonucleotide is an RNA oligonucleotide, the double-stranded nucleic acid complex is referred to as a hetero-duplex oligonucleotide. The second oligonucleotide is preferably RNA from the viewpoint of ensuring that the double-stranded nucleic acid complex is recognized by RNaseH in cells, and the second oligonucleotide is degraded to facilitate exhibition of the antisense effect of the first oligonucleotide, but the second oligonucleotide is not limited to RNA. The RNA may be modified, and may be 2-O-methylribose (2'-O-methyl-modified RNA). For example, the nucleotide complementary to the 5'-wing region and the 3'-wing region of the first oligonucleotide may be 2'-O-methyl-modified RNA.

The oligonucleotide containing a gap region, a 5'-wing region connected to the 5'-end of the gap region and a 3'-wing region connected to the 3'-end of the gap region is referred to as a gapmer. For example, an oligonucleotide in which the number of bases in the 5'-wing region is r, the number of bases in the gap region is s and the number of bases in the 3'-wing region is r is referred to as an oligonucleotide having a gapmer structure of r-s-r.

In one aspect of the present invention, the antisense strand includes a deoxyribonucleotide (DNA nucleotide) or an optionally chemically modified deoxyribonucleotide (modified DNA nucleotide). The nucleotide of the gap region may be a DNA nucleotide, or all the nucleotides may be DNA nucleotides.

In one aspect of the present invention, the second oligonucleotide having a sequence complementary to the first oligonucleotide includes a ribonucleotide (RNA nucleotide) or an optionally chemically modified ribonucleotide (modified RNA nucleotide). Some of the nucleotides may be RNA nucleotides, or all the nucleotides may be RNA nucleotides.

In the present description, the phosphorothioated internucleotide linkage, which is one form of the modified nucleotide, i.e. phosphorothioate-type DNA/RNA (i.e. DNA/RNA having a thiophosphate linkage), is referred to as a PS-modified internucleotide linkage. The phosphorothioated internucleotide linkage refers to an oligonucleotide which has a phosphodiester linkage and in which an oxygen atom of a phosphate group is replaced by a sulfur atom. The internucleotide linkage which is not phosphorothioated is referred to as a natural (PO) internucleotide linkage. Those skilled in the art can appropriately select and perform relevantly possible chemical modification according to the type of nucleotide (e.g. deoxyribonucleotide or ribonucleotide) or the type of nucleic acid analog.

PS-modified internucleotide linkages in the first oligonucleotide, particularly PS-modified internucleotide linkages in the gap region of the first oligonucleotide, are present in a certain pattern. The pattern of PS-modified internucleotide linkages is referred to as a PS modification pattern or simply as a modification pattern. Here, the PS-modified internucleotide linkage means that a linkage between a nucleotide and a nucleotide on the 3'-side thereof is phosphorothioated, and a nucleotide on the 5'-side of two nucleotides connected by the phosphorothioated internucleotide linkages is referred to as a PS-modified nucleotide.

The modification pattern of internucleotide linkages in the first oligonucleotide includes, for example, a pattern of (POPOPS)n with any internucleotide linkage added selected from PO, PS, POPO, PSPS, POPS and PSPO. Preferably, the modification pattern of internucleotide linkages in the sequence of the gap region of the first oligonucleotide is represented by, for example, a pattern of (POPOPS)n with any internucleotide linkage added selected from PO, PS, POPO, PSPS, POPS and PSPO. Here, PS represents a phosphorothioated PS-modified internucleotide linkage, PO represents a natural internucleotide linkage which is not phosphorothioated, and n represents a natural number, and is preferably 1 to 9. When the number of internucleotide linkages in the sequence of the gap region of the first oligonucleotide is p, the number of bases in the gap region of the first oligonucleotide is p + 1. For example, specifically, when the number of bases in the gap region of the oligonucleotide is 10, the number of internucleotide linkages is 9. Alternatively, the first oligonucleotide includes a pattern having m number of contiguous PS-modified internucleotide linkages from the 5'-end to the 3'-end of the first oligonucleotide (m is a natural number equal to or less than 2/3, preferably equal to or less than 1/2, of the number of nucleic acids present in the first oligonucleotide). Preferably, the internucleotide linkages in the sequence of the gap region of the first oligonucleotide have a pattern having m number of contiguous PS-modified internucleotide linkages from the 5'-end to the 3'-end of the first oligonucleotide (m is a natural number equal to or less than 2/3, preferably equal to or less than 1/2, of the number of nucleic acids present in the first oligonucleotide).

The internucleotide linkages in the 5'-wing region and the 3'-wing region of the first oligonucleotide may be PS-modified internucleotide linkages, may be PO internucleotide linkages, or may include both of PS-modified internucleotide linkages and PO internucleotide linkages. For example, one or more PS-modified internucleotide linkages may be present, or all the internucleotide linkages may be PS-modified internucleotide linkages. The internucleotide linkage on the 5'-end of the 5'-wing region and the internucleotide linkage on the 3'-end of the 3'-wing region are PS-modified internucleotide linkages.

The modification patterns for internucleotide linkages in the sequence of the first oligonucleotide, preferably from the 5'-side of the sequence of the gap region, are shown below.
POPOPS (n = 1)
POPOPSPO
POPOPSPS
POPOPSPOPO
POPOPSPSPS
POPOPSPOPS
POPOPSPSPO
POPOPSPOPOPS (n = 2)
POPOPSPOPOPSPO
POPOPSPOPOPSPS
POPOPSPOPOPSPOPO
POPOPSPOPOPSPSPS
POPOPSPOPOPSPOPS
POPOPSPOPOPSPSPO
POPOPSPOPOPSPOPOPS (n = 3)
POPOPSPOPOPSPOPOPSPO
POPOPSPOPOPSPOPOPSPS
POPOPSPOPOPSPOPOPSPOPO
POPOPSPOPOPSPOPOPSPSPS
POPOPSPOPOPSPOPOPSPOPS
POPOPSPOPOPSPOPOPSPSPO
POPOPSPOPOPSPOPOPSPOPOPS (n = 4)
POPOPSPOPOPSPOPOPSPOPOPSPO
POPOPSPOPOPSPOPOPSPOPOPSPS
POPOPSPOPOPSPOPOPSPOPOPSPOPO
POPOPSPOPOPSPOPOPSPOPOPSPSPS
POPOPSPOPOPSPOPOPSPOPOPSPOPS
POPOPSPOPOPSPOPOPSPOPOPSPSPO
POPOPSPOPOPSPOPOPSPOPOPSPOPOPS (n = 5)
POPOPSPOPOPSPOPOPSPOPOPSPOPOPSPO
POPOPSPOPOPSPOPOPSPOPOPSPOPOPSPS
POPOPSPOPOPSPOPOPSPOPOPSPOPOPSPOPO
POPOPSPOPOPSPOPOPSPOPOPSPOPOPSPSPS
POPOPSPOPOPSPOPOPSPOPOPSPOPOPSPOPS
POPOPSPOPOPSPOPOPSPOPOPSPOPOPSPSPO
POPOPSPOPOPSPOPOPSPOPOPSPOPOPSPOPOPS (n = 6)
POPOPSPOPOPSPOPOPSPOPOPSPOPOPSPOPOPSPO
POPOPSPOPOPSPOPOPSPOPOPSPOPOPSPOPOPSPS
POPOPSPOPOPSPOPOPSPOPOPSPOPOPSPOPOPSPOPO
POPOPSPOPOPSPOPOPSPOPOPSPOPOPSPOPOPSPSPS
POPOPSPOPOPSPOPOPSPOPOPSPOPOPSPOPOPSPOPS
POPOPSPOPOPSPOPOPSPOPOPSPOPOPSPOPOPSPSPO
POPOPSPOPOPSPOPOPSPOPOPSPOPOPSPOPOPSPOPOPS (n = 7)
POPOPSPOPOPSPOPOPSPOPOPSPOPOPSPOPOPSPOPOPSPO
POPOPSPOPOPSPOPOPSPOPOPSPOPOPSPOPOPSPOPOPSPS
POPOPSPOPOPSPOPOPSPOPOPSPOPOPSPOPOPSPOPOPSPOPO
POPOPSPOPOPSPOPOPSPOPOPSPOPOPSPOPOPSPOPOPSPSPS
POPOPSPOPOPSPOPOPSPOPOPSPOPOPSPOPOPSPOPOPSPOPS
POPOPSPOPOPSPOPOPSPOPOPSPOPOPSPOPOPSPOPOPSPSPO
POPOPSPOPOPSPOPOPSPOPOPSPOPOPSPOPOPSPOPOPSPOPOPS (n = 8)
POPOPSPOPOPSPOPOPSPOPOPSPOPOPSPOPOPSPOPOPSPOPOPSPO
POPOPSPOPOPSPOPOPSPOPOPSPOPOPSPOPOPSPOPOPSPOPOPSPS
POPOPSPOPOPSPOPOPSPOPOPSPOPOPSPOPOPSPOPOPSPOPOPSPOPO
POPOPSPOPOPSPOPOPSPOPOPSPOPOPSPOPOPSPOPOPSPOPOPSPSPS
POPOPSPOPOPSPOPOPSPOPOPSPOPOPSPOPOPSPOPOPSPOPOPSPOPS
POPOPSPOPOPSPOPOPSPOPOPSPOPOPSPOPOPSPOPOPSPOPOPSPSPO
POPOPSPOPOPSPOPOPSPOPOPSPOPOPSPOPOPSPOPOPSPOPOPSPOPOPS (n =9)
POPOPSPOPOPSPOPOPSPOPOPSPOPOPSPOPOPSPOPOPSPOPOPSPOPOPSPO
POPOPSPOPOPSPOPOPSPOPOPSPOPOPSPOPOPSPOPOPSPOPOPSPOPOPSPS
POPOPSPOPOPSPOPOPSPOPOPSPOPOPSPOPOPSPOPOPSPOPOPSPOPOPSPOPO
POPOPSPOPOPSPOPOPSPOPOPSPOPOPSPOPOPSPOPOPSPOPOPSPOPOPSPSPS
POPOPSPOPOPSPOPOPSPOPOPSPOPOPSPOPOPSPOPOPSPOPOPSPOPOPSPOPS
POPOPSPOPOPSPOPOPSPOPOPSPOPOPSPOPOPSPOPOPSPOPOPSPOPOPSPSPO
PSPOPOPO
PSPOPOPOPO
PSPOPOPOPOPO
PSPOPOPOPOPOPO
PSPOPOPOPOPOPOPO
PSPOPOPOPOPOPOPOPO
PSPOPOPOPOPOPOPOPOPO
PSPOPOPOPOPOPOPOPOPOPO
PSPOPOPOPOPOPOPOPOPOPOPO
PSPOPOPOPOPOPOPOPOPOPOPOPO
PSPOPOPOPOPOPOPOPOPOPOPOPOPO
PSPOPOPOPOPOPOPOPOPOPOPOPOPOPO
PSPOPOPOPOPOPOPOPOPOPOPOPOPOPOPO
PSPOPOPOPOPOPOPOPOPOPOPOPOPOPOPOPO
PSPOPOPOPOPOPOPOPOPOPOPOPOPOPOPOPOPO
PSPOPOPOPOPOPOPOPOPOPOPOPOPOPOPOPOPOPO
PSPOPOPOPOPOPOPOPOPOPOPOPOPOPOPOPOPOPOPO
PSPOPOPOPOPOPOPOPOPOPOPOPOPOPOPOPOPOPOPOPO
PSPOPOPOPOPOPOPOPOPOPOPOPOPOPOPOPOPOPOPOPOPO
PSPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPOPOPOPO
PSPSPOPO
PSPSPOPOPO
PSPSPOPOPOPO
PSPSPOPOPOPOPO
PSPSPOPOPOPOPOPO
PSPSPOPOPOPOPOPOPO
PSPSPOPOPOPOPOPOPOPO
PSPSPOPOPOPOPOPOPOPOPO
PSPSPOPOPOPOPOPOPOPOPOPO
PSPSPOPOPOPOPOPOPOPOPOPOPO
PSPSPOPOPOPOPOPOPOPOPOPOPOPO
PSPSPOPOPOPOPOPOPOPOPOPOPOPOPO
PSPSPOPOPOPOPOPOPOPOPOPOPOPOPOPO
PSPSPOPOPOPOPOPOPOPOPOPOPOPOPOPOPO
PSPSPOPOPOPOPOPOPOPOPOPOPOPOPOPOPOPO
PSPSPOPOPOPOPOPOPOPOPOPOPOPOPOPOPOPOPO
PSPSPOPOPOPOPOPOPOPOPOPOPOPOPOPOPOPOPOPO
PSPSPOPOPOPOPOPOPOPOPOPOPOPOPOPOPOPOPOPOPO
PSPSPOPOPOPOPOPOPOPOPOPOPOPOPOPOPOPOPOPOPOPO
PSPSPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPOPOPOPO
PSPSPSPOPOPO
PSPSPSPOPOPOPO
PSPSPSPOPOPOPOPO
PSPSPSPOPOPOPOPOPO
PSPSPSPOPOPOPOPOPOPO
PSPSPSPOPOPOPOPOPOPOPO
PSPSPSPOPOPOPOPOPOPOPOPO
PSPSPSPOPOPOPOPOPOPOPOPOPO
PSPSPSPOPOPOPOPOPOPOPOPOPOPO
PSPSPSPOPOPOPOPOPOPOPOPOPOPOPO
PSPSPSPOPOPOPOPOPOPOPOPOPOPOPOPO
PSPSPSPOPOPOPOPOPOPOPOPOPOPOPOPOPO
PSPSPSPOPOPOPOPOPOPOPOPOPOPOPOPOPOPO
PSPSPSPOPOPOPOPOPOPOPOPOPOPOPOPOPOPOPO
PSPSPSPOPOPOPOPOPOPOPOPOPOPOPOPOPOPOPOPO
PSPSPSPOPOPOPOPOPOPOPOPOPOPOPOPOPOPOPOPOPO
PSPSPSPOPOPOPOPOPOPOPOPOPOPOPOPOPOPOPOPOPOPO
PSPSPSPOPOPOPOPOPOPOPOPOPOPOPOPOPOPOPOPOPOPOPO
PSPSPSPOPOPOPOPOPOPOPOPOPOPOPOPOPOPOPOPOPOPOPOPO
PSPSPSPOPOPOPOPOPOPOPOPOPOPOPOPOPOPOPOPOPOPOPOPOPO
PSPSPSPOPOPOPOPOPOPOPOPOPOPOPOPOPOPOPOPOPOPOPOPOPOPO
PSPSPSPOPOPOPOPOPOPOPOPOPOPOPOPOPOPOPOPOPOPOPOPOPOPOPO
PSPSPSPSPOPOPOPO
PSPSPSPSPOPOPOPOPO
PSPSPSPSPOPOPOPOPOPO
PSPSPSPSPOPOPOPOPOPOPO
PSPSPSPSPOPOPOPOPOPOPOPO
PSPSPSPSPOPOPOPOPOPOPOPOPO
PSPSPSPSPOPOPOPOPOPOPOPOPOPO
PSPSPSPSPOPOPOPOPOPOPOPOPOPOPO
PSPSPSPSPOPOPOPOPOPOPOPOPOPOPOPO
PSPSPSPSPOPOPOPOPOPOPOPOPOPOPOPOPO
PSPSPSPSPOPOPOPOPOPOPOPOPOPOPOPOPOPO
PSPSPSPSPOPOPOPOPOPOPOPOPOPOPOPOPOPOPO
PSPSPSPSPOPOPOPOPOPOPOPOPOPOPOPOPOPOPOPO
PSPSPSPSPOPOPOPOPOPOPOPOPOPOPOPOPOPOPOPOPO
PSPSPSPSPOPOPOPOPOPOPOPOPOPOPOPOPOPOPOPOPOPO
PSPSPSPSPOPOPOPOPOPOPOPOPOPOPOPOPOPOPOPOPOPOPO
PSPSPSPSPOPOPOPOPOPOPOPOPOPOPOPOPOPOPOPOPOPOPOPO
PSPSPSPSPOPOPOPOPOPOPOPOPOPOPOPOPOPOPOPOPOPOPOPOPO
PSPSPSPSPOPOPOPOPOPOPOPOPOPOPOPOPOPOPOPOPOPOPOPOPOPO
PSPSPSPSPOPOPOPOPOPOPOPOPOPOPOPOPOPOPOPOPOPOPOPOPOPOPO
PSPSPSPSPSPOPOPOPOPO
PSPSPSPSPSPOPOPOPOPOPO
PSPSPSPSPSPOPOPOPOPOPOPO
PSPSPSPSPSPOPOPOPOPOPOPOPO
PSPSPSPSPSPOPOPOPOPOPOPOPOPO
PSPSPSPSPSPOPOPOPOPOPOPOPOPOPO
PSPSPSPSPSPOPOPOPOPOPOPOPOPOPOPO
PSPSPSPSPSPOPOPOPOPOPOPOPOPOPOPOPO
PSPSPSPSPSPOPOPOPOPOPOPOPOPOPOPOPOPO
PSPSPSPSPSPOPOPOPOPOPOPOPOPOPOPOPOPOPO
PSPSPSPSPSPOPOPOPOPOPOPOPOPOPOPOPOPOPOPO
PSPSPSPSPSPOPOPOPOPOPOPOPOPOPOPOPOPOPOPOPO
PSPSPSPSPSPOPOPOPOPOPOPOPOPOPOPOPOPOPOPOPOPO
PSPSPSPSPSPOPOPOPOPOPOPOPOPOPOPOPOPOPOPOPOPOPO
PSPSPSPSPSPOPOPOPOPOPOPOPOPOPOPOPOPOPOPOPOPOPOPO
PSPSPSPSPSPOPOPOPOPOPOPOPOPOPOPOPOPOPOPOPOPOPOPOPO
PSPSPSPSPSPOPOPOPOPOPOPOPOPOPOPOPOPOPOPOPOPOPOPOPOPO
PSPSPSPSPSPOPOPOPOPOPOPOPOPOPOPOPOPOPOPOPOPOPOPOPOPOPO
PSPSPSPSPSPSPOPOPOPOPOPOPO
PSPSPSPSPSPSPOPOPOPOPOPOPOPO
PSPSPSPSPSPSPOPOPOPOPOPOPOPOPO
PSPSPSPSPSPSPOPOPOPOPOPOPOPOPOPO
PSPSPSPSPSPSPOPOPOPOPOPOPOPOPOPOPO
PSPSPSPSPSPSPOPOPOPOPOPOPOPOPOPOPOPO
PSPSPSPSPSPSPOPOPOPOPOPOPOPOPOPOPOPOPO
PSPSPSPSPSPSPOPOPOPOPOPOPOPOPOPOPOPOPOPO
PSPSPSPSPSPSPOPOPOPOPOPOPOPOPOPOPOPOPOPOPO
PSPSPSPSPSPSPOPOPOPOPOPOPOPOPOPOPOPOPOPOPOPO
PSPSPSPSPSPSPOPOPOPOPOPOPOPOPOPOPOPOPOPOPOPOPO
PSPSPSPSPSPSPOPOPOPOPOPOPOPOPOPOPOPOPOPOPOPOPOPO
PSPSPSPSPSPSPOPOPOPOPOPOPOPOPOPOPOPOPOPOPOPOPOPOPO
PSPSPSPSPSPSPOPOPOPOPOPOPOPOPOPOPOPOPOPOPOPOPOPOPOPO
PSPSPSPSPSPSPOPOPOPOPOPOPOPOPOPOPOPOPOPOPOPOPOPOPOPOPO
PSPSPSPSPSPSPSPOPOPOPOPOPOPOPO
PSPSPSPSPSPSPSPOPOPOPOPOPOPOPOPO
PSPSPSPSPSPSPSPOPOPOPOPOPOPOPOPOPO
PSPSPSPSPSPSPSPOPOPOPOPOPOPOPOPOPOPO
PSPSPSPSPSPSPSPOPOPOPOPOPOPOPOPOPOPOPO
PSPSPSPSPSPSPSPOPOPOPOPOPOPOPOPOPOPOPOPO
PSPSPSPSPSPSPSPOPOPOPOPOPOPOPOPOPOPOPOPOPO
PSPSPSPSPSPSPSPOPOPOPOPOPOPOPOPOPOPOPOPOPOPO
PSPSPSPSPSPSPSPOPOPOPOPOPOPOPOPOPOPOPOPOPOPOPO
PSPSPSPSPSPSPSPOPOPOPOPOPOPOPOPOPOPOPOPOPOPOPOPO
PSPSPSPSPSPSPSPOPOPOPOPOPOPOPOPOPOPOPOPOPOPOPOPOPO
PSPSPSPSPSPSPSPOPOPOPOPOPOPOPOPOPOPOPOPOPOPOPOPOPOPO
PSPSPSPSPSPSPSPOPOPOPOPOPOPOPOPOPOPOPOPOPOPOPOPOPOPOPO
PSPSPSPSPSPSPSPSPOPOPOPOPOPOPOPOPO
PSPSPSPSPSPSPSPSPOPOPOPOPOPOPOPOPOPO
PSPSPSPSPSPSPSPSPOPOPOPOPOPOPOPOPOPOPO
PSPSPSPSPSPSPSPSPOPOPOPOPOPOPOPOPOPOPOPO
PSPSPSPSPSPSPSPSPOPOPOPOPOPOPOPOPOPOPOPOPO
PSPSPSPSPSPSPSPSPOPOPOPOPOPOPOPOPOPOPOPOPOPO
PSPSPSPSPSPSPSPSPOPOPOPOPOPOPOPOPOPOPOPOPOPOPO
PSPSPSPSPSPSPSPSPOPOPOPOPOPOPOPOPOPOPOPOPOPOPOPO
PSPSPSPSPSPSPSPSPOPOPOPOPOPOPOPOPOPOPOPOPOPOPOPOPO
PSPSPSPSPSPSPSPSPOPOPOPOPOPOPOPOPOPOPOPOPOPOPOPOPOPO
PSPSPSPSPSPSPSPSPOPOPOPOPOPOPOPOPOPOPOPOPOPOPOPOPOPOPO
PSPSPSPSPSPSPSPSPSPOPOPOPOPOPOPOPOPOPO
PSPSPSPSPSPSPSPSPSPOPOPOPOPOPOPOPOPOPOPO
PSPSPSPSPSPSPSPSPSPOPOPOPOPOPOPOPOPOPOPOPO
PSPSPSPSPSPSPSPSPSPOPOPOPOPOPOPOPOPOPOPOPOPO
PSPSPSPSPSPSPSPSPSPOPOPOPOPOPOPOPOPOPOPOPOPOPO
PSPSPSPSPSPSPSPSPSPOPOPOPOPOPOPOPOPOPOPOPOPOPOPO
PSPSPSPSPSPSPSPSPSPOPOPOPOPOPOPOPOPOPOPOPOPOPOPOPO
PSPSPSPSPSPSPSPSPSPOPOPOPOPOPOPOPOPOPOPOPOPOPOPOPOPO
PSPSPSPSPSPSPSPSPSPOPOPOPOPOPOPOPOPOPOPOPOPOPOPOPOPOPO
PSPSPSPSPSPSPSPSPSPSPOPOPOPOPOPOPOPOPOPOPO
PSPSPSPSPSPSPSPSPSPSPOPOPOPOPOPOPOPOPOPOPOPO
PSPSPSPSPSPSPSPSPSPSPOPOPOPOPOPOPOPOPOPOPOPOPO
PSPSPSPSPSPSPSPSPSPSPOPOPOPOPOPOPOPOPOPOPOPOPOPO
PSPSPSPSPSPSPSPSPSPSPOPOPOPOPOPOPOPOPOPOPOPOPOPOPO
PSPSPSPSPSPSPSPSPSPSPOPOPOPOPOPOPOPOPOPOPOPOPOPOPOPO
PSPSPSPSPSPSPSPSPSPSPOPOPOPOPOPOPOPOPOPOPOPOPOPOPOPOPO
PSPSPSPSPSPSPSPSPSPSPSPOPOPOPOPOPOPOPOPOPOPOPO
PSPSPSPSPSPSPSPSPSPSPSPOPOPOPOPOPOPOPOPOPOPOPOPO
PSPSPSPSPSPSPSPSPSPSPSPOPOPOPOPOPOPOPOPOPOPOPOPOPO
PSPSPSPSPSPSPSPSPSPSPSPOPOPOPOPOPOPOPOPOPOPOPOPOPOPO
PSPSPSPSPSPSPSPSPSPSPSPOPOPOPOPOPOPOPOPOPOPOPOPOPOPOPO
PSPSPSPSPSPSPSPSPSPSPSPSPOPOPOPOPOPOPOPOPOPOPOPOPO
PSPSPSPSPSPSPSPSPSPSPSPSPOPOPOPOPOPOPOPOPOPOPOPOPOPO
PSPSPSPSPSPSPSPSPSPSPSPSPOPOPOPOPOPOPOPOPOPOPOPOPOPOPO
PSPSPSPSPSPSPSPSPSPSPSPSPSPOPOPOPOPOPOPOPOPOPOPOPOPOPO

In a double-stranded nucleic acid complex in a certain embodiment, a functional portion may be bound to the second oligonucleotide. The linkage between the second oligonucleotide and the functional portion may be a direct linkage, or an indirect linkage through another substance. In an embodiment, it is preferable that the second oligonucleotide be directly bound to the functional portion via a covalent bond, an ionic bond, a hydrogen bond or the like, and a covalent bond is more preferable from the viewpoint of obtaining a more stable linkage.

In an embodiment, the structure of the "functional portion" is not particularly limited, and a desired function is imparted to a double-stranded nucleic acid complex and/or a nucleic acid strand to which the functional portion is bound. Examples of the desired function include a labeling function, a purification function and a function of delivery to a target. Examples of the portion that imparts a labeling function include compounds such as fluorescent proteins and luciferase. Examples of the portion that imparts a purification function include compounds such as biotin, avidin, His-tagged peptide, GST-tagged peptide and FLAG-tagged peptide.

From the viewpoint of efficiently delivering the first oligonucleotide to a target site with high specificity and very effectively suppressing expression of a target gene by the nucleic acid, it is preferable that molecules having activity that delivers a double-stranded nucleic acid complex in a certain embodiment to the target site be bound to the second oligonucleotide as a functional portion.

Examples of the portion of the "function of delivery to a target" include lipids from the viewpoint of being able to efficiently deliver a double-stranded nucleic acid complex in an embodiment to the liver or the like with high specificity. Examples of the lipids include cholesterol, lipids such as fatty acids (e.g. vitamin E (tocopherols and tocotrienols), vitamin A and vitamin D), fat-soluble vitamins such as vitamin k (e.g. acyl carnitine), intermediate metabolites such as acyl CoA, glycolipids, glycerides, and derivatives thereof, and of these, cholesterol and vitamin E (tocopherols and tocotrienols) are preferably used in an embodiment from the viewpoint of higher safety. Examples of the "functional portion" in an embodiment include sugars (e.g. glucose and sucrose) from the viewpoint of being able to efficiently deliver the double-stranded nucleic acid of the present invention to the brain with high specificity. Examples of the "functional portion" in an embodiment include peptides or proteins such as ligands and antibodies for receptors and/or fragments thereof from the viewpoint of being able to efficiently deliver a double-stranded nucleic acid complex in an embodiment to each organ by binding to various proteins on cell surfaces of the organ with high specificity.

A double-stranded nucleic acid complex in some embodiments is efficiently delivered to a target site with high specificity and can very effectively suppress expression of a target gene or a target transcription product level as shown in Examples described below. Therefore, the present invention can provide a composition which contains a double-stranded nucleic acid complex in some embodiments as an active ingredient and is intended to, for example, suppress expression of a target gene by an antisense effect. In particular, a double-stranded nucleic acid complex in some embodiments enables high drug efficacy to be obtained by administration at a low concentration and also enables reduction of side effects by suppressing the distribution of an antisense nucleic acid in organs other than a delivery target region, and therefore a pharmaceutical composition for treating or preventing diseases associated with increased expression of a target gene, such as metabolic diseases, tumors and infections, can be provided in some embodiments.

A composition containing a double-stranded nucleic acid complex in some embodiments can be formulated by a known pharmaceutical method. The composition can be used enterally (e.g. orally) or non-enterally as, for example, capsules, tablets, pills, solutions, powders, granules, subtle granules, film coating preparations, pellets, troches, sublingual preparations, masticatories, buccals, pastes, syrups, suspensions, elixirs, emulsions, embrocations, ointments, plasters, cataplasms, transdermal absorption preparations, lotions, inhalants, aerosols, injections and suppositories.

For such formulation, the composition can be appropriately combined with carriers acceptable pharmacologically or as a food or beverage product, specifically sterilized water, physiological saline, vegetable oil, solvents, bases, emulsifiers, suspending agents, surfactants, pH regulators, stabilizers, flavoring agents, fragrances, excipients, vehicles, preservatives, binders, diluents, tonicity agents, soothing agents, extenders, disintegrants, buffering agents, coatings, lubricants, colorants, sweeteners, thickening agents, taste and odor correctives, solubilizers or other additives.

In a double-stranded nucleic acid complex in some embodiments, a complex with a lipoprotein such as chylomicron or chylomicron remnant may be formed. Further, from the viewpoint of enhancing the efficiency of enteric administration, a complex (mixed micelle or emulsion) with a substance having a large-intestinal mucosa epithelial permeability increasing action (e.g. a medium-chain fatty acid, a long-chain unsaturated fatty acid or a derivative (a salt, an ester or an ether) thereof) and a surfactant (a nonionic surfactant or an anionic surfactant) in addition to the lipoprotein may be formed.

The preferred dosage form of a composition in some embodiments is not particularly limited, and examples thereof include enteric (oral) or non-enteric administration, more specifically intravenous administration, intraarterial administration, intraperitoneal administration, subcutaneous administration, intradermal administration, intratracheal administration, rectal administration, intramuscular administration, and administration by transfusion.

A composition in some embodiments can be used for animals including humans, animals other than humans are not particularly limited, and various live stocks, poultries, pets, experimental animals and the like may be subjects.

When a composition in some embodiments is administered or ingested, the dose or intake thereof is appropriately selected according to the age, the body weight, the symptom or the health condition of a subject, or the type of the composition (e.g. medicament or food or beverage product), and the effective intake of a composition according to an embodiment is preferably 0.001 mg/kg/day to 50 mg/kg/day in terms of nucleotide.

A double-stranded nucleic acid in some embodiments is efficiently delivered to a target site with high specificity and can very effectively suppress expression of a target gene or a target transcription product level as shown in Examples described below. Therefore, some embodiments can provide a method in which a double-stranded nucleic acid complex in some embodiments is administered to a subject to suppress expression of a target gene or a target transcription product by an antisense effect. It is also possible to provide a method for treating or preventing various diseases associated with increased expression of a target gene by administering a composition in some embodiments to a subject.

### Examples

The present invention will be described in detail by way of Examples below, which should not be construed as limiting the present invention.

Even when among PS (phosphorothioation modification)-introduced sites in internucleotide linkages at 13 positions on an antisense strand which is a first oligonucleotide including a gapmer structure of a heteronucleic acid having a strand length of 14 mer, PSs at 6 positions were turned to a natural type (PO), there were found 2 patterns of PS-introduced sites where effectivity did not decrease at 7 positions. Accordingly, avoidance of a side effect as a tendency to bleed in the administration acute stage and improvement in bioavailability in subcutaneous administration were achieved.

In the present invention, 7 types of heteroduplex oligonucleotides (HDOs) selected from the PTEN gene, having a gapmer structure of 2-10-2 and including antisense strands having different patterns of PS modification were designed. In the sense strands which are second oligonucleotides of these HDOs, the wing portion contained 2'-O-methyl-modified RNA and RNA was disposed in the gap region. Further, the inter-nucleic acid linkages at 2 positions from each of both ends of the sense strand were subjected to PS modification, and cholesterol (Chol) was added to the 5'-end as a ligand. The synthesis of the oligo was commissioned to Gene design Inc.

**[Table 1]**

| Sequence of PTEN HDO | | | | |
|---|---|---|---|---|
| Nº | Designation | | Added to 5'-end | Sequence (5' → 3') |
| 1 | PTEN-1 | Antisense strand | | T(L)^5(L)^a^t^g^g^c^t^g^c^a^g^5(L)^T(L) |
| | | Sense strand | Chol | A(M)^G(M)^CUGCAGCCAU^G(M)^A(M) |
| 2 | PTEN-2 | Antisense strand | | T(L)^5(L)a^t^g^g^c^t^g^c^a^g5(L)^T(L) |
| | | Sense strand | Chol | A(M)^G(M)^CUGCAGCCAU^G(M)^A(M) |
| 3 | PTEN-3 | Antisense strand | | T(L)^5(L)^atggctg^c^a^g^5(L)^T(L) |
| | | Sense strand | Chol | A(M)^G(M)^CUGCAGCCAU^G(M)^A(M) |
| 4 | PTEN-4 | Antisense strand | | T(L)^5(L)^a^t^g^gctgcag^5(L)^T(L) |
| | | Sense strand | Chol | A(M)^G(M)^CUGCAGCCAU^G(M)^A(M) |
| 5 | PTEN-5 | Antisense strand | | T(L)^5(L)^atg^gct^gca^g^5(L)^T(L) |
| | | Sense strand | Chol | A(M)^G(M)^CUGCAGCCAU^G(M)^A(M) |
| 6 | PTEN-6 | Antisense strand | | T(L)^5(L)^a^t^gg^c^tg^c^ag^5(L)^T(L) |
| | | Sense strand | Chol | A(M)^G(M)^CUGCAGCCAU^G(M)^A(M) |
| 7 | PTEN-7 | Antisense strand | | T(L)^5(L)^at^gg^ct^gc^ag^5(L)^T(L) |
| | | Sense strand | Chol | A(M)^G(M)^CUGCAGCCAU^G(M)^A(M) |

The lowercase character represents DNA, the uppercase character represents RNA, N(L) represents a LNA modification [e.g. A(L), T(L), G(L), mC (LNA methylcytosine) is 5(L)], Λ (chevron mark) represents a PS-modified internucleotide linkage, the uppercase character (M) represents 2'-O-methyl-modified RNA [e.g. A(M), G(M), C(M), U(M)], and Chol represents cholesterol.

The sequences of the antisense strands of 1 to 7 in the table above are the sequences of SEQ ID NOs: 1 to 7, respectively, and the sequence of the sense strand is the sequence of SEQ ID NO: 8 in the sequence listing.

The PS modifications in the internucleotide linkages at 13 positions in the antisense strands of the 7 types of HDOs were designed as follows. In the present invention, the internucleotide linkages in the gap region, i.e. the PS modification patterns for the third to eleventh internucleotide linkages below, are important.

PTEN-1 PsPs PsPsPsPsPsPsPsPsPs PsPs (Ps:PO = 13:0)
PTEN-2 PsPO PsPsPsPsPsPsPsPsPs POPs (Ps:PO = 11:2)
PTEN-3 PsPs POPOPOPOPOPOPsPsPs PsPs (Ps:PO = 7:6)
PTEN-4 PsPs PsPsPsPOPOPOPOPOPO PsPs (Ps:PO = 7:6)
PTEN-5 PsPs POPOPsPOPOPsPOPOPs PsPs (Ps:PO = 7:6)
PTEN-6 PsPs PsPsPOPsPsPOPsPsPO PsPs (Ps:PO = 10:3)
PTEN-7 PsPO PsPsPOPsPsPOPsPsPO POPs (Ps:PO = 8:5)

### [Example 1]

### Comparison of Knockdown Action in Liver When Seven Types of PTEN HDOs (14 mer) Having Different PS Modification Patterns for Antisense Strands are Administered to Mouse

The knockdown action in the liver when 7 types of PTEN HDOs (14 mer) having different PS modification patterns for antisense strands were administered to a c57BL/6 mouse was examined. Vehicle (saline) was used as a negative control, and all-PS-modified PTEN-1 was used as a positive control. The dose of PTEN HDO was set to 100 nmol/kg, and the PTEN HDO was administered in a single dose (10 ml/kg) through the tail vein of the mouse. On day 3 after the administration, the mouse was subjected to intracardiac blood collection under isoflurane anesthesia, and the liver was isolated. Total RNA was extracted from the liver tissues using SV Total RNA Isolation System (Promega KK), a reverse transcription reaction was carried out using PrimeScript (registered trademark) RT Master Mix (Takara Bio, Inc.), and quantitative PCR was then carried out using LightCycler (registered trademark) 480 System II (F. Hoffmann-La Roche, Ltd.). The reagents from TaqMan Gene Expression Assays (Thermo Fisher Scientific, Inc.) were used as primers and probes for PTEN and 18SrRNA, and the mRNA expression level was calculated by the ΔΔCt method.

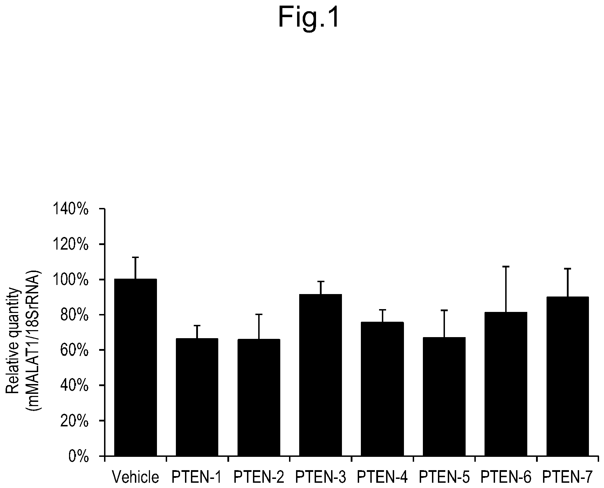

As shown in Figure 1, PTEN-4 in which only three inter-nucleic acid linkages from the 5'-end in the gap region were PS-modified (7 PSs) showed a knockdown action at 24% of that of Vehicle. PTEN-5 having a repetitive sequence of three POPOPS modifications in the gap region (7 PSs) showed a knockdown action at 33% of that of the Vehicle administration group. The knockdown rate of PTEN-5 was equivalent to that of all-PS-modified PTEN-1 (13 PSs). It is very interesting that PTEN-5 showed higher knockdown activity over PTEN-6 and PTEN-7 having a repetitive sequence of three PSPSPO modification patterns. The above results revealed that PTEN-5 can have a smaller number of PS modifications without decreasing knockdown activity as compared to PTEN-1.

### [Example 2]

### Change in Liver Weight When 7 Types of PTEN HDOs (14 mer) Having Different PS Modification Patterns for Antisense Strands are Administered to Mouse

Since PTEN-4 and PTEN-5 showed knockdown activity in Example 1, whether or not a side effect as toxicity occurred at 100 nmol/kg, i.e. the same dose of PTEN HDO as in Example 1, was determined.

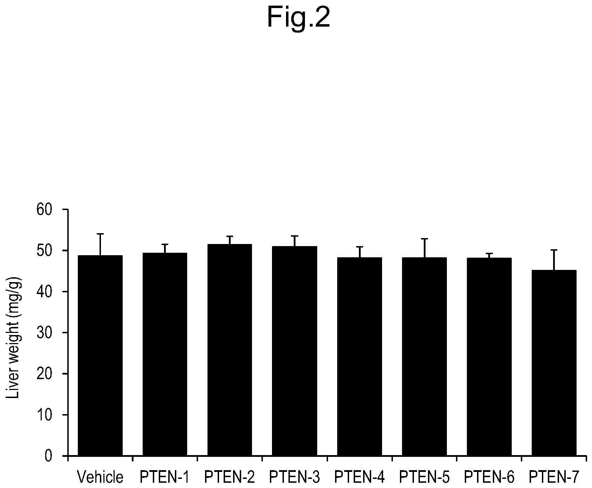

A mouse was subjected to the treatment described in Example 1. 3 days after the administration, the mouse was anesthetized with isoflurane, and then subjected to intracardiac blood collection using a syringe treated with heparin sodium in advance, and the liver was then isolated. Figure 2 shows changes in mouse liver weight 3 days after 7 types of PTEN HDOs (14 mer) having different PS modification patterns for antisense strands are administered. There was no significant difference in liver weight between the Vehicle administration group and each of the PTEN HDO administration groups regardless of the PS modification pattern.

### [Example 3]

### Change in Blood ALT Activity When 7 Types of PTEN HDOs (14 mer) Having Different PS Modification Patterns for Antisense Strands are Administered to Mouse

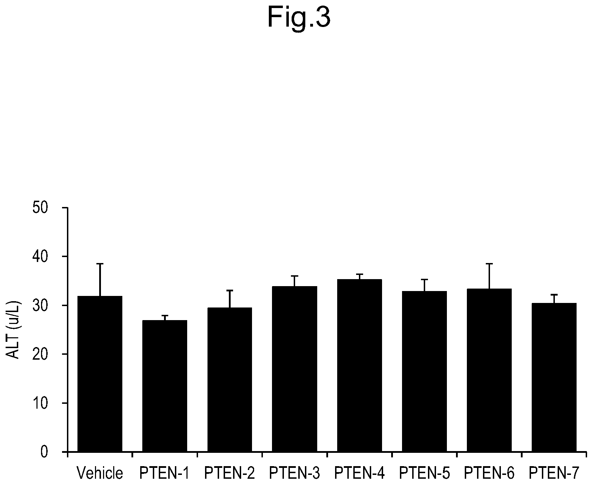

A mouse was subjected to the treatment described in Example 1. 3 days after the administration, the mouse was anesthetized with isoflurane, and then subjected to intracardiac blood collection using a syringe treated with heparin sodium in advance, and the liver was then isolated. The blood was centrifuged, and using the supernatant, blood liver deviation enzyme (ALT) activity was measured with Transaminase CII-Test Wako (FUJIFILM Wako Pure Chemical Corporation). Figure 3 shows changes in blood ALT activity of the mouse 3 days after 7 types of PTEN HDOs (14 mer) having different PS modification patterns for antisense strands are administered. There was no significant difference in blood ALT activity between the Vehicle administration group and each of the PTEN HDO administration groups regardless of the PS modification pattern.

### [Example 4]

### Effectivity of Patterns Different in Gene and Strand Length from PTEN-1

In this Example, three types of hetero-duplex oligonucleotides (HDOs) selected from MALAT1 and ApoB genes, having a 3-10-3 gapmer structure and including antisense strands having different PS modification patterns, i.e. an all-PS-modification and two types of patterns having different PS modification patterns: (POPOPS)n and m number of contiguous PS-modified nucleotides from the 5'-end (m is equal to or less than 2/3, preferably equal to or less than 1/2, of the strand length of the antisense strand), were designed. In the sense strands of these HDOs, the wing portion contained 2'-O-methyl-modified RNA and RNA was disposed in the gap region. Further, the inter-nucleic acid linkages at 3 positions from each of both ends of the sense strand were subjected to PS modification, and cholesterol (Chol) was added to the 5'-end as a ligand. The synthesis of the oligo was commissioned to Gene design Inc.

**[Table 2]**

| Sequences of MALAT1 and ApoB HDO | | | | |
|---|---|---|---|---|
| Nº | Designation | | Added to 5'-end | Sequence (5' → 3') |
| 1 | MALAT1 | Antisense strand | | 5(L)^T(L)^A(L)^g^t^t^c^a^c^t^g^a^a^T(L)^G (L)^5(L) |
| | | Sense strand | Chol | G(M)^C(M)^A(M)^UUCAGUGAAC^U(M)^A(M)^G(M) |
| 2 | ApoB | Antisense strand | | 5(L)^A(L)^G(L)^c^a^t^t^g^g^t^a^t^t^5(L)^A (L)^G(L) |
| | | Sense strand | Chol | C(M)^U(M)^G(M)^AAUACCAAUG^C(M)^U(M)^G(M) |

In Table 2, n represents DNA, N represents RNA, N(L) represents a LNA modification [A(L), T(L), G(L), mC is 5(L)], Λ represents a PS modification, N(M) represents 2'-O-methyl-modified RNA [A(M), G(M), C(M), U(M)], and Chol represents cholesterol.

The sequences of the antisense strand and the sense strand of MALAT1 in Table 2 are set forth as SEQ ID NOs: 9 and 10, respectively, in the sequence listing, and the sequences of the antisense strand and the sense strand of ApoB are set forth as SEQ ID NOs: 11 and 12, respectively, in the sequence listing.

PS modifications in three types of 3-10-3 gapmers, the antisense strands of HDOs, and the antisense strands of single-stranded antisense oligomers (ASOs) selected from MALAT1 genes and ApoB genes were designed as follows.

MALAT1(1) PsPsPs PsPsPsPsPsPsPsPsPs PsPsPs (Ps:PO = 15:0)
MALAT1(2) PsPsPs PsPsPsPoPoPoPoPoPo PsPsPs (Ps:Po = 9:6)
MALAT1(3) PsPsPs PoPoPsPoPoPsPoPoPs PsPsPs (Ps:Po = 9:6)
ApoB (1) PsPsPs PsPsPsPsPsPsPsPsPs PsPsPs (Ps:PO = 15:0)
ApoB (2) PsPsPs PsPsPsPoPoPoPoPoPo PsPsPs (Ps:Po = 9:6)
ApoB (3) PsPsPs PoPoPsPoPoPsPoPoPs PsPsPs (Ps:Po = 9:6)

### [Example 5]

### 1) Knockdown Action of Two Types of HDOs Having Different PS Modification Patterns for Antisense Strand of MALAT1

The knockdown action of MALAT1 HDO (MALAT1(2) and MALAT1(3), 16 mer) on MALAT1 mRNA of the liver was examined using a c57BL/6 mouse. Vehicle (saline) was used as a negative control, and all-PS-modified MLAT1(1) was used as a positive control. The dose of MALAT1 HDO was set to 100 nmol/kg, the mouse was anesthetized with isoflurane, and the MALAT1 HDO was then administered in a single dose (10 ml/kg) through the tail vein. 3 days after the administration, total RNA was extracted from the mouse liver using SV Total RNA Isolation System (Promega KK), a reverse transcription reaction was carried out using PrimeScript RT Master Mix (Takara Bio, Inc.), and quantitative PCR was then carried out using LightCycler (registered trademark) 480 System II (F. Hoffmann-La Roche, Ltd.). The reagents from TaqMan Gene Expression Assays (Thermo Fisher Scientific, Inc.) were used as primers and probes for qPCR detection, and the mRNA expression level was calculated by the ΔΔCt method.

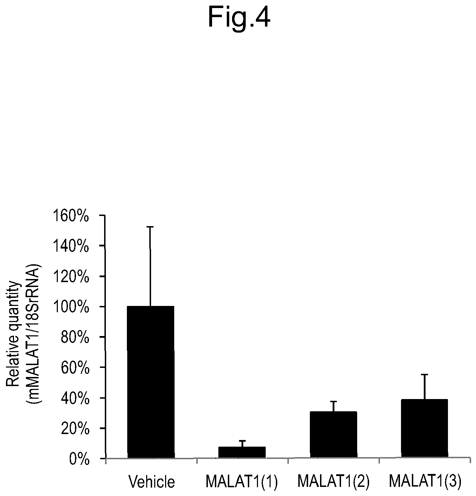

As shown in Figure 4, the all-PS-modified MALAT1(1) (15 PSs) showed the highest knockdown activity (93%). In contrast, MALAT1(2) and MALAT1(3) (9 PSs) suppressed MALAT1 mRNA expression in the liver by 70% and 62%, respectively.

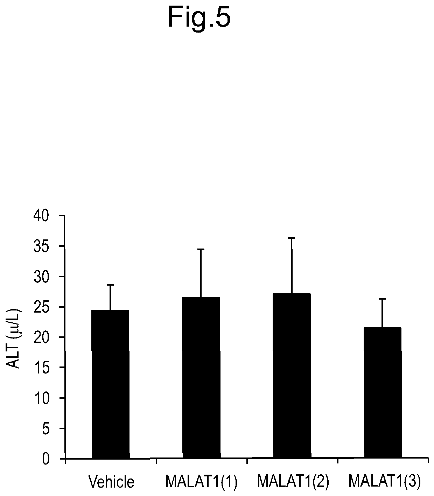

In any of the MALAT1 HDO administration groups, the blood ALT activity 3 days after the administration of MALAT1 HDO was not significantly different from that in the Vehicle administration group. Figure 5 shows the ALT activity measurement results.

### [Example 6]

### Knockdown Action of 2 Types of HDOs (16 mer) Having Different PS Modification Patterns for Antisense Strand of ApoB

The knockdown action of ApoB HDO (ApoB(2) and ApoB(3), 16 mer) on ApoB mRNA expression in the liver was examined using a c57BL/6 mouse. Vehicle (saline) was used as a negative control, and all-PS-modified ApoB(1) was used as a positive control. The dose of ApoB HDO was set to 100 nmol/kg, the mouse was anesthetized with isoflurane, and the ApoB HDO was then administered in a single dose (10 ml/kg) through the tail vein. 3 days after the administration, total RNA was extracted from the mouse liver using SV Total RNA Isolation System (Promega KK), a reverse transcription reaction was carried out using PrimeScript RT Master Mix (Takara Bio, Inc.), and quantitative PCR was then carried out using LightCycler (registered trademark) 480 System II (F. Hoffmann-La Roche, Ltd.). The reagents from TaqMan Gene Expression Assays (Thermo Fisher Scientific, Inc.) were used as primers and probes for qPCR detection, and the mRNA expression level was calculated by the ΔΔCt method.

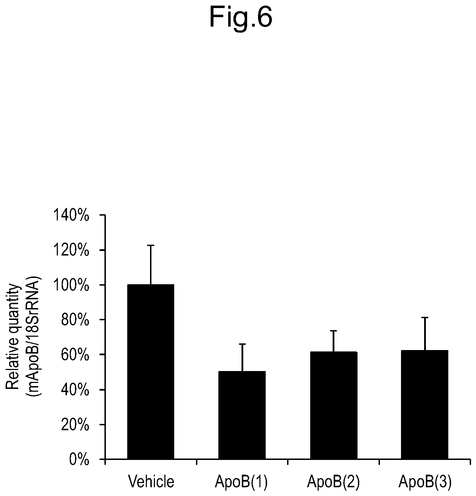

As shown in Figure 6, the all-PS-modified ApoB(1) (15 PSs) knocked down ApoB mRNA expression in the liver by 50%. In contrast, ApoB(2) and ApoB(3) (9 PSs) suppressed ApoB mRNA expression in the liver by 39% and 38%, respectively.

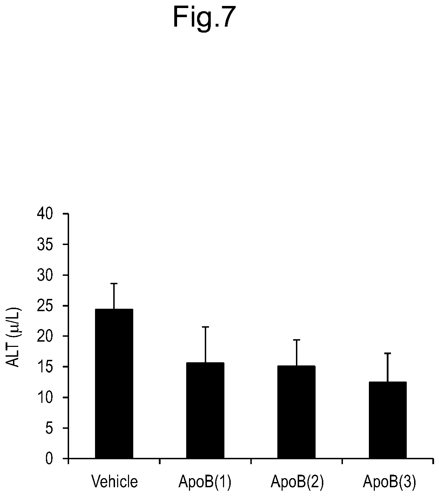

In any of the ApoB HDO administration groups, the blood ALT activity 3 days after the administration of ApoB HDO was not significantly different from that in the Vehicle administration group. Figure 7 shows the ALT activity measurement results.

### [Example 7]

### Blood Coagulation Time Reducing Effect of 2 Types of HDOs Having Different PS Modification Patterns on Extension of Blood Coagulation Time by All-PS-Modified Oligo

The effect of the PS-modified oligo on the tendency to bleed in the administration acute stage was examined by use of activated partial thromboplastin time (APTT). All-PS-modified MALAT1 ASO (16 mer, single strand) was used as a positive control. The same number of test tubes as the number of blood plasma samples were prepared, and 0.1 mL of an APTT reagent was taken into each test tube, and heated at 37°C for 1 minute. Next, 0.1 mL of each of a blood plasma sample from a normal mouse and blood plasma samples from mice given ASO, MALAT1(1), HDO MALAT1(1), (2) and (3), and HDO ApoB(1), (2) and (3) was added and mixed, and the mixture was heated at 37°C for 2 minutes. Next, the activated partial thromboplastin time (aPTT) of plasma adjusted by adding 0.1 mL of a calcium chloride solution heated to 37°C in advance to each tube was measured using an automatic blood coagulation measuring apparatus CA-50 (Sysmex Corporation).

Table 3 shows the results. The APTT of the untreated blood (normal) was 24.3 seconds. On the other hand, the APTT with all-PS-modified MALAT1 HDO was 59.7 seconds, and each of the APTT times with MALAT1(2) and MALAT1(3) having a smaller number of PS modifications was 48.6 seconds, and shorter by about 20% than the APTT with all-PS-modified MALAT1 HDO. The APTT with all-PS-modified ApoB(1) was 55.3 seconds, and the APTT times with ApoB(2) and ApoB(3) were 45.6 seconds and 45.8 seconds, respectively, and shorter by about 20% than the APTT with all-PS-modified ApoB(1). The APTT with all-PS-modified MALAT1 ASO(1) as a positive control was 75.8 seconds, and about 3 times longer than the APTT of the untreated blood (normal), which was 24.3 seconds. The APTT is an index such that the standard value (normal value) is 25.0 to 40.0 seconds, and the possibility of liver function disorder increases as the APTT becomes farther from the standard value. Each of the APTT times with ApoB(2) and ApoB(3) was shorter than the APTT time with ApoB(1), that is, liver function disorder which is a side effect as a tendency to bleed in the administration acute stage was improved.

**[Table 3]**

| Effect on APTT | | | | |
|---|---|---|---|---|
| | (Amount equivalent to 5 mg/kg) | | No. | APTT (sec) |
| Plasma | - | - | - | 24.3 |
| ASO | MALAT1 | 16 mer | (1) | 75.8 |
| HDO | MALAT1 | 16 mer | (1) | 59.7 |
| | MALAT1 | 16 mer | (2) | 48.6 |
| | MALAT1 | 16 mer | (3) | 48.6 |
| | ApoB | 16 mer | (1) | 55.3 |
| | ApoB | 16 mer | (2) | 45.6 |
| | ApoB | 16 mer | (3) | 45.8 |

In Examples 4, 5, 6 and 7, some of PSs in each of MALAT1(1) and ApoB(2) which are all-PS-modified patterns containing PSs at 15 positions in an antisense strand including the gapmer structure of a heteronucleic acid having a strand length of 16 mer were changed to POs to obtain 2 types of patterns: MALAT1(2) and MALAT1(3) and ApoB(2) and ApoB(3), which did not cause a decrease in effectivity and shortened the APTT time. Accordingly, avoidance of a side effect as a tendency to bleed in the administration acute stage and improvement in bioavailability in subcutaneous administration were achieved.

### Industrial Applicability

The double-stranded nucleic acid complex of the present invention can be used as a nucleic acid drug.

### Sequence Listing Free Text

### SEQ ID NOs: 1 to 12 Synthetic

All publications, patents and patent applications cited in the present description are incorporated herein by reference in their entirety.

## Claims

1. A double-stranded nucleic acid complex comprising: a first oligonucleotide comprising an antisense strand consisting of 7 to 30 nucleotides; and a second oligonucleotide having a sequence complementary to the first oligonucleotide and hybridized to the first oligonucleotide, wherein a modification pattern of internucleotide linkages in the first oligonucleotide comprises a pattern of (POPOPS)n with any internucleotide linkage added selected from PO, PS, POPO, PSPS, POPS and PSPO [PS represents a phosphorothioated PS-modified internucleotide linkage, PO represents a non-phosphorothioated natural internucleotide linkage, and n represents a natural number] or a pattern having m number of contiguous PS-modified internucleotide linkages from the 5'-end to the 3'-end of the first oligonucleotide (m is a natural number equal to or less than 2/3 of the number of nucleic acids present in the first oligonucleotide).

2. The double-stranded nucleic acid complex according to claim 1, comprising: a first oligonucleotide comprising an antisense strand consisting of 7 to 30 nucleotides; and a second oligonucleotide having a sequence complementary to the first oligonucleotide and hybridized to the first oligonucleotide, wherein the modification pattern of internucleotide linkages in the first oligonucleotide comprises a pattern of (POPOPS)n with any internucleotide linkage added selected from PO, PS, POPO, PSPS, POPS and PSPO [PS represents a phosphorothioated PS-modified internucleotide linkage, PO represents a non-phosphorothioated natural internucleotide linkage, and n is 1 to 9].

3. The double-stranded nucleic acid complex according to claim 2, wherein the modification pattern of internucleotide linkages in the first oligonucleotide is any of the following:
POPOPS (n = 1)
POPOPSPO
POPOPSPS
POPOPSPOPO
POPOPSPSPS
POPOPSPOPS
POPOPSPSPO
POPOPSPOPOPS (n = 2)
POPOPSPOPOPSPO
POPOPSPOPOPSPS
POPOPSPOPOPSPOPO
POPOPSPOPOPSPSPS
POPOPSPOPOPSPOPS
POPOPSPOPOPSPSPO
POPOPSPOPOPSPOPOPS (n = 3)
POPOPSPOPOPSPOPOPSPO
POPOPSPOPOPSPOPOPSPS
POPOPSPOPOPSPOPOPSPOPO
POPOPSPOPOPSPOPOPSPSPS
POPOPSPOPOPSPOPOPSPOPS
POPOPSPOPOPSPOPOPSPSPO
POPOPSPOPOPSPOPOPSPOPOPS (n = 4)
POPOPSPOPOPSPOPOPSPOPOPSPO
POPOPSPOPOPSPOPOPSPOPOPSPS
POPOPSPOPOPSPOPOPSPOPOPSPOPO
POPOPSPOPOPSPOPOPSPOPOPSPSPS
POPOPSPOPOPSPOPOPSPOPOPSPOPS
POPOPSPOPOPSPOPOPSPOPOPSPSPO
POPOPSPOPOPSPOPOPSPOPOPSPOPOPS (n = 5)
POPOPSPOPOPSPOPOPSPOPOPSPOPOPSPO
POPOPSPOPOPSPOPOPSPOPOPSPOPOPSPS
POPOPSPOPOPSPOPOPSPOPOPSPOPOPSPOPO
POPOPSPOPOPSPOPOPSPOPOPSPOPOPSPSPS
POPOPSPOPOPSPOPOPSPOPOPSPOPOPSPOPS
POPOPSPOPOPSPOPOPSPOPOPSPOPOPSPSPO
POPOPSPOPOPSPOPOPSPOPOPSPOPOPSPOPOPS (n = 6)
POPOPSPOPOPSPOPOPSPOPOPSPOPOPSPOPOPSPO
POPOPSPOPOPSPOPOPSPOPOPSPOPOPSPOPOPSPS
POPOPSPOPOPSPOPOPSPOPOPSPOPOPSPOPOPSPOPO
POPOPSPOPOPSPOPOPSPOPOPSPOPOPSPOPOPSPSPS
POPOPSPOPOPSPOPOPSPOPOPSPOPOPSPOPOPSPOPS
POPOPSPOPOPSPOPOPSPOPOPSPOPOPSPOPOPSPSPO
POPOPSPOPOPSPOPOPSPOPOPSPOPOPSPOPOPSPOPOPS (n = 7)
POPOPSPOPOPSPOPOPSPOPOPSPOPOPSPOPOPSPOPOPSPO
POPOPSPOPOPSPOPOPSPOPOPSPOPOPSPOPOPSPOPOPSPS
POPOPSPOPOPSPOPOPSPOPOPSPOPOPSPOPOPSPOPOPSPOPO
POPOPSPOPOPSPOPOPSPOPOPSPOPOPSPOPOPSPOPOPSPSPS
POPOPSPOPOPSPOPOPSPOPOPSPOPOPSPOPOPSPOPOPSPOPS
POPOPSPOPOPSPOPOPSPOPOPSPOPOPSPOPOPSPOPOPSPSPO
POPOPSPOPOPSPOPOPSPOPOPSPOPOPSPOPOPSPOPOPSPOPOPS (n = 8)
POPOPSPOPOPSPOPOPSPOPOPSPOPOPSPOPOPSPOPOPSPOPOPSPO
POPOPSPOPOPSPOPOPSPOPOPSPOPOPSPOPOPSPOPOPSPOPOPSPS
POPOPSPOPOPSPOPOPSPOPOPSPOPOPSPOPOPSPOPOPSPOPOPSPOPO
POPOPSPOPOPSPOPOPSPOPOPSPOPOPSPOPOPSPOPOPSPOPOPSPSPS
POPOPSPOPOPSPOPOPSPOPOPSPOPOPSPOPOPSPOPOPSPOPOPSPOPS
POPOPSPOPOPSPOPOPSPOPOPSPOPOPSPOPOPSPOPOPSPOPOPSPSPO
POPOPSPOPOPSPOPOPSPOPOPSPOPOPSPOPOPSPOPOPSPOPOPSPOPOPS (n = 9)
POPOPSPOPOPSPOPOPSPOPOPSPOPOPSPOPOPSPOPOPSPOPOPSPOPOPSPO
POPOPSPOPOPSPOPOPSPOPOPSPOPOPSPOPOPSPOPOPSPOPOPSPOPOPSPS
POPOPSPOPOPSPOPOPSPOPOPSPOPOPSPOPOPSPOPOPSPOPOPSPOPOPSPOPO
POPOPSPOPOPSPOPOPSPOPOPSPOPOPSPOPOPSPOPOPSPOPOPSPOPOPSPSPS
POPOPSPOPOPSPOPOPSPOPOPSPOPOPSPOPOPSPOPOPSPOPOPSPOPOPSPOPS
POPOPSPOPOPSPOPOPSPOPOPSPOPOPSPOPOPSPOPOPSPOPOPSPOPOPSPSPO.

4. The double-stranded nucleic acid complex according to claim 3, wherein the modification pattern of internucleotide linkages in the first oligonucleotide is POPOPSPOPOPSPOPOPS.

5. A double-stranded nucleic acid complex comprising: a first oligonucleotide comprising an antisense strand consisting of 7 to 30 nucleotides; and a second oligonucleotide having a sequence complementary to the first oligonucleotide and hybridized to the first oligonucleotide, wherein a modification pattern of internucleotide linkages in the first oligonucleotide comprises a pattern having m number of contiguous PS-modified internucleotide linkages from the 5'-end to the 3'-end of the first oligonucleotide (m is a natural number equal to or less than 2/3 of the number of nucleic acids present in the first oligonucleotide).

6. A double-stranded nucleic acid complex comprising: a first oligonucleotide comprising an antisense strand consisting of 7 to 28 nucleotides; and a second oligonucleotide having a sequence complementary to the first oligonucleotide and hybridized to the first oligonucleotide, wherein a modification pattern of internucleotide linkages in the first oligonucleotide comprises a pattern having m number of contiguous PS-modified internucleotide linkages from the 5'-end to the 3'-end of the first oligonucleotide (m is a natural number equal to or less than 2/3 of the number of nucleic acids present in the first oligonucleotide, which is in the range of 1 to 13).

7. The double-stranded nucleic acid complex according to claim 6, wherein the modification pattern of internucleotide linkages in the first oligonucleotide is any of the following:
PSPOPOPO
PSPOPOPOPO
PSPOPOPOPOPO
PSPOPOPOPOPOPO
PSPOPOPOPOPOPOPO
PSPOPOPOPOPOPOPOPO
PSPOPOPOPOPOPOPOPOPO
PSPOPOPOPOPOPOPOPOPOPO
PSPOPOPOPOPOPOPOPOPOPOPO
PSPOPOPOPOPOPOPOPOPOPOPOPO
PSPOPOPOPOPOPOPOPOPOPOPOPOPO
PSPOPOPOPOPOPOPOPOPOPOPOPOPOPO
PSPOPOPOPOPOPOPOPOPOPOPOPOPOPOPO
PSPOPOPOPOPOPOPOPOPOPOPOPOPOPOPOPO
PSPOPOPOPOPOPOPOPOPOPOPOPOPOPOPOPOPO
PSPOPOPOPOPOPOPOPOPOPOPOPOPOPOPOPOPOPO
PSPOPOPOPOPOPOPOPOPOPOPOPOPOPOPOPOPOPOPO
PSPOPOPOPOPOPOPOPOPOPOPOPOPOPOPOPOPOPOPOPO
PSPOPOPOPOPOPOPOPOPOPOPOPOPOPOPOPOPOPOPOPOPO
PSPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPOPOPO
PSPOPOPOPOPOPOPOPOPOPOPOPOPOPOPOPOPOPOPOPOPOPOPOPOPOPO
PSPSPOPO
PSPSPOPOPO
PSPSPOPOPOPO
PSPSPOPOPOPOPO
PSPSPOPOPOPOPOPO
PSPSPOPOPOPOPOPOPO
PSPSPOPOPOPOPOPOPOPO
PSPSPOPOPOPOPOPOPOPOPO
PSPSPOPOPOPOPOPOPOPOPOPO
PSPSPOPOPOPOPOPOPOPOPOPOPO
PSPSPOPOPOPOPOPOPOPOPOPOPOPO
PSPSPOPOPOPOPOPOPOPOPOPOPOPOPO
PSPSPOPOPOPOPOPOPOPOPOPOPOPOPOPO
PSPSPOPOPOPOPOPOPOPOPOPOPOPOPOPOPO
PSPSPOPOPOPOPOPOPOPOPOPOPOPOPOPOPOPO
PSPSPOPOPOPOPOPOPOPOPOPOPOPOPOPOPOPOPO
PSPSPOPOPOPOPOPOPOPOPOPOPOPOPOPOPOPOPOPO
PSPSPOPOPOPOPOPOPOPOPOPOPOPOPOPOPOPOPOPOPO
PSPSPOPOPOPOPOPOPOPOPOPOPOPOPOPOPOPOPOPOPOPO
PSPSPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPOPOPO
PSPSPOPOPOPOPOPOPOPOPOPOPOPOPOPOPOPOPOPOPOPOPOPOPOPOPO
PSPSPSPOPOPO
PSPSPSPOPOPOPO
PSPSPSPOPOPOPOPO
PSPSPSPOPOPOPOPOPO
PSPSPSPOPOPOPOPOPOPO
PSPSPSPOPOPOPOPOPOPOPO
PSPSPSPOPOPOPOPOPOPOPOPO
PSPSPSPOPOPOPOPOPOPOPOPOPO
PSPSPSPOPOPOPOPOPOPOPOPOPOPO
PSPSPSPOPOPOPOPOPOPOPOPOPOPOPO
PSPSPSPOPOPOPOPOPOPOPOPOPOPOPOPO
PSPSPSPOPOPOPOPOPOPOPOPOPOPOPOPOPO
PSPSPSPOPOPOPOPOPOPOPOPOPOPOPOPOPOPO
PSPSPSPOPOPOPOPOPOPOPOPOPOPOPOPOPOPOPO
PSPSPSPOPOPOPOPOPOPOPOPOPOPOPOPOPOPOPOPO
PSPSPSPOPOPOPOPOPOPOPOPOPOPOPOPOPOPOPOPOPO
PSPSPSPOPOPOPOPOPOPOPOPOPOPOPOPOPOPOPOPOPOPO
PSPSPSPOPOPOPOPOPOPOPOPOPOPOPOPOPOPOPOPOPOPOPO
PSPSPSPOPOPOPOPOPOPOPOPOPOPOPOPOPOPOPOPOPOPOPOPO
PSPSPSPOPOPOPOPOPOPOPOPOPOPOPOPOPOPOPOPOPOPOPOPOPO
PSPSPSPOPOPOPOPOPOPOPOPOPOPOPOPOPOPOPOPOPOPOPOPOPOPO
PSPSPSPOPOPOPOPOPOPOPOPOPOPOPOPOPOPOPOPOPOPOPOPOPOPOPO
PSPSPSPSPOPOPOPO
PSPSPSPSPOPOPOPOPO
PSPSPSPSPOPOPOPOPOPO
PSPSPSPSPOPOPOPOPOPOPO
PSPSPSPSPOPOPOPOPOPOPOPO
PSPSPSPSPOPOPOPOPOPOPOPOPO
PSPSPSPSPOPOPOPOPOPOPOPOPOPO
PSPSPSPSPOPOPOPOPOPOPOPOPOPOPO
PSPSPSPSPOPOPOPOPOPOPOPOPOPOPOPO
PSPSPSPSPOPOPOPOPOPOPOPOPOPOPOPOPO
PSPSPSPSPOPOPOPOPOPOPOPOPOPOPOPOPOPO
PSPSPSPSPOPOPOPOPOPOPOPOPOPOPOPOPOPOPO
PSPSPSPSPOPOPOPOPOPOPOPOPOPOPOPOPOPOPOPO
PSPSPSPSPOPOPOPOPOPOPOPOPOPOPOPOPOPOPOPOPO
PSPSPSPSPOPOPOPOPOPOPOPOPOPOPOPOPOPOPOPOPOPO
PSPSPSPSPOPOPOPOPOPOPOPOPOPOPOPOPOPOPOPOPOPOPO
PSPSPSPSPOPOPOPOPOPOPOPOPOPOPOPOPOPOPOPOPOPOPOPO
PSPSPSPSPOPOPOPOPOPOPOPOPOPOPOPOPOPOPOPOPOPOPOPOPO
PSPSPSPSPOPOPOPOPOPOPOPOPOPOPOPOPOPOPOPOPOPOPOPOPOPO
PSPSPSPSPOPOPOPOPOPOPOPOPOPOPOPOPOPOPOPOPOPOPOPOPOPOPO
PSPSPSPSPSPOPOPOPOPO
PSPSPSPSPSPOPOPOPOPOPO
PSPSPSPSPSPOPOPOPOPOPOPO
PSPSPSPSPSPOPOPOPOPOPOPOPO
PSPSPSPSPSPOPOPOPOPOPOPOPOPO
PSPSPSPSPSPOPOPOPOPOPOPOPOPOPO
PSPSPSPSPSPOPOPOPOPOPOPOPOPOPOPO
PSPSPSPSPSPOPOPOPOPOPOPOPOPOPOPOPO
PSPSPSPSPSPOPOPOPOPOPOPOPOPOPOPOPOPO
PSPSPSPSPSPOPOPOPOPOPOPOPOPOPOPOPOPOPO
PSPSPSPSPSPOPOPOPOPOPOPOPOPOPOPOPOPOPOPO
PSPSPSPSPSPOPOPOPOPOPOPOPOPOPOPOPOPOPOPOPO
PSPSPSPSPSPOPOPOPOPOPOPOPOPOPOPOPOPOPOPOPOPO
PSPSPSPSPSPOPOPOPOPOPOPOPOPOPOPOPOPOPOPOPOPOPO
PSPSPSPSPSPOPOPOPOPOPOPOPOPOPOPOPOPOPOPOPOPOPOPO
PSPSPSPSPSPOPOPOPOPOPOPOPOPOPOPOPOPOPOPOPOPOPOPOPO
PSPSPSPSPSPOPOPOPOPOPOPOPOPOPOPOPOPOPOPOPOPOPOPOPOPO
PSPSPSPSPSPOPOPOPOPOPOPOPOPOPOPOPOPOPOPOPOPOPOPOPOPOPO
PSPSPSPSPSPSPOPOPOPOPOPOPO
PSPSPSPSPSPSPOPOPOPOPOPOPOPO
PSPSPSPSPSPSPOPOPOPOPOPOPOPOPO
PSPSPSPSPSPSPOPOPOPOPOPOPOPOPOPO
PSPSPSPSPSPSPOPOPOPOPOPOPOPOPOPOPO
PSPSPSPSPSPSPOPOPOPOPOPOPOPOPOPOPOPO
PSPSPSPSPSPSPOPOPOPOPOPOPOPOPOPOPOPOPO
PSPSPSPSPSPSPOPOPOPOPOPOPOPOPOPOPOPOPOPO
PSPSPSPSPSPSPOPOPOPOPOPOPOPOPOPOPOPOPOPOPO
PSPSPSPSPSPSPOPOPOPOPOPOPOPOPOPOPOPOPOPOPOPO
PSPSPSPSPSPSPOPOPOPOPOPOPOPOPOPOPOPOPOPOPOPOPO
PSPSPSPSPSPSPOPOPOPOPOPOPOPOPOPOPOPOPOPOPOPOPOPO
PSPSPSPSPSPSPOPOPOPOPOPOPOPOPOPOPOPOPOPOPOPOPOPOPO
PSPSPSPSPSPSPOPOPOPOPOPOPOPOPOPOPOPOPOPOPOPOPOPOPOPO
PSPSPSPSPSPSPOPOPOPOPOPOPOPOPOPOPOPOPOPOPOPOPOPOPOPOPO
PSPSPSPSPSPSPSPOPOPOPOPOPOPOPO
PSPSPSPSPSPSPSPOPOPOPOPOPOPOPOPO
PSPSPSPSPSPSPSPOPOPOPOPOPOPOPOPOPO
PSPSPSPSPSPSPSPOPOPOPOPOPOPOPOPOPOPO
PSPSPSPSPSPSPSPOPOPOPOPOPOPOPOPOPOPOPO
PSPSPSPSPSPSPSPOPOPOPOPOPOPOPOPOPOPOPOPO
PSPSPSPSPSPSPSPOPOPOPOPOPOPOPOPOPOPOPOPOPO
PSPSPSPSPSPSPSPOPOPOPOPOPOPOPOPOPOPOPOPOPOPO
PSPSPSPSPSPSPSPOPOPOPOPOPOPOPOPOPOPOPOPOPOPOPO
PSPSPSPSPSPSPSPOPOPOPOPOPOPOPOPOPOPOPOPOPOPOPOPO
PSPSPSPSPSPSPSPOPOPOPOPOPOPOPOPOPOPOPOPOPOPOPOPOPO
PSPSPSPSPSPSPSPOPOPOPOPOPOPOPOPOPOPOPOPOPOPOPOPOPOPO
PSPSPSPSPSPSPSPOPOPOPOPOPOPOPOPOPOPOPOPOPOPOPOPOPOPOPO
PSPSPSPSPSPSPSPSPOPOPOPOPOPOPOPOPO
PSPSPSPSPSPSPSPSPOPOPOPOPOPOPOPOPOPO
PSPSPSPSPSPSPSPSPOPOPOPOPOPOPOPOPOPOPO
PSPSPSPSPSPSPSPSPOPOPOPOPOPOPOPOPOPOPOPO
PSPSPSPSPSPSPSPSPOPOPOPOPOPOPOPOPOPOPOPOPO
PSPSPSPSPSPSPSPSPOPOPOPOPOPOPOPOPOPOPOPOPOPO
PSPSPSPSPSPSPSPSPOPOPOPOPOPOPOPOPOPOPOPOPOPOPO
PSPSPSPSPSPSPSPSPOPOPOPOPOPOPOPOPOPOPOPOPOPOPOPO
PSPSPSPSPSPSPSPSPOPOPOPOPOPOPOPOPOPOPOPOPOPOPOPOPO
PSPSPSPSPSPSPSPSPOPOPOPOPOPOPOPOPOPOPOPOPOPOPOPOPOPO
PSPSPSPSPSPSPSPSPOPOPOPOPOPOPOPOPOPOPOPOPOPOPOPOPOPOPO
PSPSPSPSPSPSPSPSPSPOPOPOPOPOPOPOPOPOPO
PSPSPSPSPSPSPSPSPSPOPOPOPOPOPOPOPOPOPOPO
PSPSPSPSPSPSPSPSPSPOPOPOPOPOPOPOPOPOPOPOPO
PSPSPSPSPSPSPSPSPSPOPOPOPOPOPOPOPOPOPOPOPOPO
PSPSPSPSPSPSPSPSPSPOPOPOPOPOPOPOPOPOPOPOPOPOPO
PSPSPSPSPSPSPSPSPSPOPOPOPOPOPOPOPOPOPOPOPOPOPOPO
PSPSPSPSPSPSPSPSPSPOPOPOPOPOPOPOPOPOPOPOPOPOPOPOPO
PSPSPSPSPSPSPSPSPSPOPOPOPOPOPOPOPOPOPOPOPOPOPOPOPOPO
PSPSPSPSPSPSPSPSPSPOPOPOPOPOPOPOPOPOPOPOPOPOPOPOPOPOPO
PSPSPSPSPSPSPSPSPSPSPOPOPOPOPOPOPOPOPOPOPO
PSPSPSPSPSPSPSPSPSPSPOPOPOPOPOPOPOPOPOPOPOPO
PSPSPSPSPSPSPSPSPSPSPOPOPOPOPOPOPOPOPOPOPOPOPO
PSPSPSPSPSPSPSPSPSPSPOPOPOPOPOPOPOPOPOPOPOPOPOPO
PSPSPSPSPSPSPSPSPSPSPOPOPOPOPOPOPOPOPOPOPOPOPOPOPO
PSPSPSPSPSPSPSPSPSPSPOPOPOPOPOPOPOPOPOPOPOPOPOPOPOPO
PSPSPSPSPSPSPSPSPSPSPOPOPOPOPOPOPOPOPOPOPOPOPOPOPOPOPO
PSPSPSPSPSPSPSPSPSPSPSPOPOPOPOPOPOPOPOPOPOPOPO
PSPSPSPSPSPSPSPSPSPSPSPOPOPOPOPOPOPOPOPOPOPOPOPO
PSPSPSPSPSPSPSPSPSPSPSPOPOPOPOPOPOPOPOPOPOPOPOPOPO
PSPSPSPSPSPSPSPSPSPSPSPOPOPOPOPOPOPOPOPOPOPOPOPOPOPO
PSPSPSPSPSPSPSPSPSPSPSPOPOPOPOPOPOPOPOPOPOPOPOPOPOPOPO
PSPSPSPSPSPSPSPSPSPSPSPSPOPOPOPOPOPOPOPOPOPOPOPOPO
PSPSPSPSPSPSPSPSPSPSPSPSPOPOPOPOPOPOPOPOPOPOPOPOPOPO
PSPSPSPSPSPSPSPSPSPSPSPSPOPOPOPOPOPOPOPOPOPOPOPOPOPOPO
PSPSPSPSPSPSPSPSPSPSPSPSPSPOPOPOPOPOPOPOPOPOPOPOPOPOPO.

8. The double-stranded nucleic acid complex according to claim 7, wherein the modification pattern of internucleotide linkages in the first oligonucleotide is PsPsPsPOPOPOPOPOPO.

9. The double-stranded nucleic acid complex according to any one of claims 1 to 8, wherein the first oligonucleotide includes a gap region and a wing region located at each of both ends of the gap region, and the modification pattern of internucleotide linkages in the first oligonucleotide is a modification pattern of internucleotide linkages in the gap region.

10. The double-stranded nucleic acid complex according to any one of claims 1 to 9, wherein a ligand is bound to the second oligonucleotide.

11. The double-stranded nucleic acid complex according to any one of claims 1 to 10, wherein the wing region of the first oligonucleotide includes 1 to 4 nucleic acids.

12. The double-stranded nucleic acid complex according to any one of claims 1 to 11, wherein the first oligonucleotide has a structure represented by (first wing region)r-(gap region)s-(second wing region)r [r is 1 to 10 and s is 4 to 28].

13. The double-stranded nucleic acid complex according to any one of claims 1 to 12, wherein the wing region of the first oligonucleotide comprises one or more PS-modified internucleotide linkages.

14. The double-stranded nucleic acid complex according to any one of claims 1 to 12, wherein all the internucleotide linkages in the wing region of the first oligonucleotide are PS-modified internucleotide linkages.

15. The double-stranded nucleic acid complex according to any one of claims 1 to 12, wherein the internucleotide linkage on the 5'-end of a first wing region of the first oligonucleotide and the internucleotide linkage on the 3'-end of a second wing region are PS-modified internucleotide linkages.

16. The double-stranded nucleic acid complex according to any one of claims 1 to 15, wherein the gap region of the first oligonucleotide includes DNA or a DNA-modified nucleotide, and the second oligonucleotide includes RNA or a RNA-modified nucleotide.
